# EUROPEAN PATENT SPECIFICATION

(11) **EP 3 631 009 B1**
(45) Date of publication and mention of the grant of the patent: **10.11.2021**
(21) Application number: 18727020.2
(22) Date of filing: 01.06.2018
(51) Int. Cl.: C12Q 1/6834, C12Q 1/6869, B01D 67/00, B01D 69/10, B01D 69/12, C12Q 1/6825

(54) **IMMOBILISATION OF NUCLEIC ACIDS ON SURFACES**
IMMOBILISIERUNG VON NUKLEINSÄUREN AUF OBERFLÄCHEN
IMMOBILISATION D'ACIDES NUCLÉIQUES SUR DES SURFACES

(30) Priority: 02.06.2017 LU 100276
(43) Date of publication of application: 08.04.2020
(73) Proprietor: CNM Technologies GmbH, 33615 Bielefeld (DE); Aptarion Biotech, 10589 Berlin (DE)
(72) Inventor: SCHNIEDERS, Albert, 33602 Bielefeld (DE); MEYERBRÖKER, Nikolaus, 33602 Bielefeld (DE); VATER, Axel, 13467 Berlin (DE); MAASCH, Christian, 13509 Berlin (DE); BETHGE, D. Lucas, 14482 Potsdam (DE)
(74) Representative: Harrison, Robert John
(86) International application number: PCT/EP2018/064457
(87) International publication number: WO 2018/220173

(56) References cited:
- WO-A1-2017/072272
- US-A1- 2011 184 196
- US-B2- 8 911 852
- JI XIAO-TANG ET AL: "Azide-functionalization of carbon nanotubes by electrochemical oxidation of N3-in", CHINESE CHEMICAL LETTERS, ELSEVIER, AMSTERDAM, NL, vol. 25, no. 2, 22 November 2013 (2013-11-22), pages 292-294, XP028829078, ISSN: 1001-8417, DOI: 10.1016/J.CCLET.2013.11.023
- ANDREY TURCHANIN ET AL: "Carbon Nanomembranes", ADVANCED MATERIALS, vol. 28, no. 29, 1 August 2016 (2016-08-01), pages 6075-6103, XP055438228, DE ISSN: 0935-9648, DOI: 10.1002/adma.201506058
- RADOVAN VUKICEVIC ET AL: "Alkyne-azide coupling of tailored poly(vinylidene fluoride) and polystyrene for the synthesis of block copolymers", POLYMER CHEMISTRY, vol. 3, no. 2, 1 January 2012 (2012-01-01) , pages 409-414, XP055437980, GB ISSN: 1759-9954, DOI: 10.1039/C1PY00427A
- POLINA ANGELOVA ET AL: "A Universal Scheme to Convert Aromatic Molecular Monolayers into Functional Carbon Nanomembranes", ACS NANO, vol. 7, no. 8, 27 August 2013 (2013-08-27) , pages 6489-6497, XP055236230, US ISSN: 1936-0851, DOI: 10.1021/nn402652f

## Description

### Funding

This project has received funding from the European Union's Horizon 2020 research and innovation program under grant agreement No. 634415.

### Field of the Invention

The present invention relates to the immobilisation of nucleic acids or other biomolecules on surfaces which are suitable amongst other purposes for bio-sensors.

### Background of the invention

Biosensors are commonly used for the detection or identification of molecules of various origins. The reliable identification of a target molecule, the availability of a suitable biological recognition element and the potential for disposable portable detection systems can be regarded as the most important requirements for biosensors. A specific application for biosensors is analyte detection and analytic monitoring in vivo. The biosensor may be partially or completely implanted for such a purpose. This requires biocompatibility of all materials of the biosensor including a biological recognition element.

The sensitive biological element of biosensors, or receptor, (e.g. tissue, microorganisms, organelles, cell receptors, enzymes, antibodies, nucleic acids, etc.) is a biologically derived material or biomimetic component that interacts (binds or recognizes) with the analyte under study conditions. The biologically sensitive elements can also be created by biological engineering. The transducer or the detector element (works in a physicochemical way; optical, piezoelectric, electrochemical, etc.) transforms the signal resulting from the interaction of the analyte with the biological element into another signal (i.e., transduction) that can be more easily measured and quantified. Readers are usually custom-designed and manufactured to suit the different working principles of biosensors.

Attaching biological elements like small molecules having a molecular weight below 900 daltons, proteins or cells to the surface of the sensor can be regarded as one of the most important steps, independent from the material of which the surface of the biosensor is made, including metal, polymer, carbon or glass. One of the easiest ways is to functionalise the surface in order to coat it with a biological element. Functionalisation can be achieved by using poly-lysine, aminosilane, epoxysilane or nitrocellulose in case of silicon chips or silica glass. Subsequently, the bound biological agent may be fixed for example by layer-by-layer deposition of alternately charged polymer coatings. Alternatively, three-dimensional lattices (hydrogel/xerogel) can be used to chemically or physically entrap the biological element. Chemically entrapped means that the biological element is kept in place by a strong bond, while physically entrapped means that they are kept in place by preventing to pass through the pores of the gel matrix.

Functionalisation of a surface does further depend on the class of receptor that shall be attached and the type of sensor principle that will be used. Many label-free biosensors rely on a surface-near interaction, e.g. within an evanescent field which is present on the surface. The closer the biological receptor is bound onto the surface, the more sensitive an analyte binding event can be recorded. Yet, surface proximity often compromises the functionality of biological receptor molecules. Important sensor principles based on surface-near interactions comprise reflectometric interference spectroscopy (RifS), Mach-Zehnder interferometer, guided-mode resonance (grating coupler), dynamic biosensors (switchable nanolevers), surface plasmon resonance (SPR), field effect transistor (such as Graphene-based field effect transistors (GapheneFET)), cantilever-based biosensors and ellipsometry.

A decisive step in the development of biosensors and microarrays is a successful surface tethering of sensitive biological elements such as nucleic acids and probes for detection and/or identification of analytes. In contrast to homogeneous assays, heterogeneous systems offer rapid, continuous, and reusable monitoring and allow miniaturisation. Nucleic acids (NA) have been established as one of the most important classes for such bio-sensitive elements. NA-biosensors are generally based on immobilisation of single-stranded DNA (ssDNA) or RNA (ssRNA), and they recognise a complementary target sequence by hybridisation or by adopting a three-dimensional structure that allows specific ligand interaction. [Review articles: Sipova et al., Anal. Chim. Acta., 773 (2013) 9 and Sassolas et al., Chem. Rev., 108 (2008) 109].

An important application of immobilised NAs other than microarrays is massive parallel sequencing. Depending on the method, the sequence of a single nucleic acid molecule (e.g. Helicos sequencer), a repetitive single molecule (e.g. BGISEQ-500) or a whole (monoclonal) cluster of identical molecules is identified by an iterative sequencing process. The latter is currently commercially applied in sequencing platforms available from Illumina (HiSeq, MiSeq, etc.), Thermo Fisher Scientific (SOLiD, IonTorrent), Genapsys (GENIUS), BGI (BGISEQ-500) and Qiagen (GeneReader). In order to identify sequences with high quality and quantity, the generation of monoclonal clusters must be highly efficient. Initial experiments using typical means for direct attachment of nucleic acids to glass surfaces were not successful as the anchoring was not sufficiently stable [Adessi et al., Nucleic Acids Res., 28 (2000) E87]. So far, primer oligonucleotides for cluster generation have mostly been immobilised by copolymerisation in a hydrogel. However, different clusters in a three-dimensional hydrogel may not be exactly in the same focal plane therefore hampering optimal detection. In addition, the hydrogel on the surface may also bind non-incorporated labelled nucleotides causing a background that is highly problematic especially in the setting of single molecule sequencing.

Other functional oligonucleotides such as aptamers (target-binding single-stranded oligonucleotides that often include unnatural nucleotides) are an extremely versatile class of probes. Specific aptamers can be selected in vitro to virtually any given target molecule. The selection procedure ensures extraordinarily high selectivity and affinity of an aptamer to molecules, even those with comparatively low molecular weight (e.g. drug agents, lipids, hormones, chemokines).

The immobilisation of the desired oligonucleotides to the sensor surface represents a crucial step and is usually carried out using short bifunctional molecules (linkers) that react on one end with the surface of the sensor and the other end can covalently bind a functionalised oligonucleotide. Such linkers must be biocompatible to avoid non-specific adsorption when detecting an analyte in complex real-life samples such as blood or mucus. It is a known issue that oligonucleotides in proximity to a surface are often related to a loss of their active conformation due to the interaction with the surface. Thus, target-recognition that relies on an intact three-dimensional structure will be lost. In essence, the choice of an appropriate spacer and surface is highly sophisticated and crucial for a sensitive and specific biosensor.

Many techniques have been developed to immobilise oligonucleotides on sensor surfaces. [Review: Balamurugan et al., Anal. Bioanal. Chem., 390 (2008) 1009] Besides those relying on ionic or physical interaction that would inevitably interfere with proper folding of aptamers and thereby with their target recognition, nucleic acid immobilisation by covalent attachment is by far most widely used. Comparably high grafting densities of up to 4 x 10¹³ molecules per cm² can be achieved with direct coupling of thiolated probes on gold surfaces as self-assembled monolayers (SAMs). However, this approach is limited to a few surface materials (substrates) and the oligonucleotides still readily interact with the surface. [Review: Balamurugan et al., Anal. Bioanal. Chem., 390 (2008) 1009]. In addition, molecules immobilised to gold surfaces by thiol groups slowly detach from the surface under flow conditions.

Many other immobilisation schemes like those relying on nucleophilic-electrophilic reactions are susceptible to side reactions, for instance, with amine groups of the oligonucleotides' nucleobases, with hydroxyl groups on the sugar moiety or with small molecule contaminants inherent to oligonucleotide synthesis. Additionally, popular reactive electrophiles, such as N-hydroxysuccinimide esters, are prone to hydrolysis before and during the coupling reaction, which both reduce coupling yields and can make the yield irreproducible. [Devaraj, J. Am. Chem. Soc., 127 (2005) 8600]. The authors suggest copper-induced click chemistry. However, copper, being a bivalent ion has been found to lead to NA hydrolysis. Instead, copper-free click chemistry for immobilisation of oligonucleotides on surfaces can be used [Eeftens et al., BMC Biophys., 8 (2015) 9].

US Patent Application Publication US 2011/184196 A1 teaches a carbon surface modified with one or more azide groups.

Carbon nanomembranes (CNMs) are novel two-dimensional (2D) carbon-based materials produced from radiation-induced crosslinking of a layer of precursor molecules with an aromatic molecular backbone. CNMs based on self-assembled monolayers (SAMs) are disclosed in U.S. Patent No. US 6,764,758 B1 and by Turchanin and Gölzhäuser (Adv. Mater., 28 (2016) 6075). Despite their thicknesses of only 0.3 to 30 nm, CNMs are exceptional mechanically, chemically and thermally stable. These unique properties are derived from the high degree of lateral cross-linking since they are not observed on non-cross-linked layers of precursor molecules.

A CNM can be manufactured by the following steps:
1. Providing a substrate.
2. Applying a layer of a low-molecular aromatic species (precursors). Preferably the layer is a monolayer. In case the precursor molecules carry a head group suitable to the substrate, the layer is preferably a self-assembled monolayer.
3. Exposure of the layer with radiation - preferably low energy electrons but also x-ray radiation, β-radiation, γ-radiation, photons, ions or plasma such that the molecules in the layer are crosslinked in the lateral direction to form the final CNM.

Optionally, the initial substrate can be removed to obtain a freestanding CNM, which can be transferred to nearly any other support.

In case of a SAM, the use of molecular precursors with additional end groups leads initially to monolayers with these groups at the SAM-ambient interface. After crosslinking, the result is a CNM with defined surface functionality, which does not necessarily need to be identical to those of the SAM. For example, terminal nitro groups of a SAM are reduced during the crosslinking process into reactive amine groups making the CNM accessible for electrophilic reagents, opening almost an unlimited possibility of varying a CNM.

Therefore, it is possible to bind further nanoscale objects, such as macromolecules, functionalised nanoparticles or proteins to amine-terminated CNMs as disclosed in US patent applications US 20160093806 A1 or International application WO 2016/050492 A1. It is further possible to manufacture a CNM having a pattern of functional groups (comp. US 8,911,852 B2). A typical underivatised CNM is amorphous and consists substantially but not exclusively of carbon-carbon bonds. Therefore, a CNM does not contain any charged functional groups and can be considered neutral.

The published International Application WO 2017/072272 A1 discloses a method for the manufacture of a carbon nanomembrane. The method comprises preparing a metallised polymer substrate and applying on the metallised polymer substrate a monolayer prepared from an aromatic molecule. The aromatic molecule is cross-linked to form a carbon nanomembrane. The carbon nanomembrane is coated by a protective layer and subsequently the carbon nanomembrane and the protective layer are released from the metallised polymer substrate. Finally, the carbon nanomembrane and the protective layer are optionally placed on a support. The protective layer can be optionally removed. The carbon nanomembrane can be used for filtration.

In contrast to graphene oxide (GO) - a widely used 2D-material for chemical functionalisation applications - functional groups in CNMs are well-defined, enabling specific functionalisation of CNMs even differently on their opposite faces. [Zheng et al., Angew. Chem., 122 (2010) 8671]. Additionally, GO is a flake 2D-material, therefore a GO surface coating exhibits a thickness of many molecular layers, whereas a CNMs is a molecularly thin single-layer sheet material.

Many biosensors require receptors to be stably immobilised close to the surface for high sensitivity. However, the surface strongly influences structure and activity of immobilised receptors. Especially, any weak form of interaction with the surface already compromises the function of highly structured oligonucleotides such as aptamers, severely limiting their use in biosensors. So far, no surface chemistry is available that can immobilise aptamers at high density close to the surface while retaining their functionality. Furthermore, derivatisation of surfaces with organic solvents is often not compatible with typical hardware of biosensors like plastic. Many surfaces cannot be modified with functional groups at all.

Thus, there is a need to provide a material for immobilisation of receptors like oligonucleotides, which is thin enough so that binding events to the receptors can interact with surface-sensitive sensors.

### Summary of the invention

The invention is defined by the appended claims.

The present disclosure provides a method for the manufacture of a functionalised carbon nanomembrane (CNM) comprising the steps of providing a surface and low-molecular weight aromatic precursor molecules comprising either amine groups or at least one of nitrile and nitro groups; applying a layer of the precursor molecules on the surface; crosslinking the layer of the precursor molecules in lateral direction by exposure to radiation for forming the CNM wherein nitrile or nitro groups are reduced to amine groups; and reacting the amine groups with a linker comprising at least one azide group resulting in an azide-terminated CNM.

The amine group of the CNM can be reacted with the azide group-containing linker molecule by a strong amine-reactive group.

It is intended that for crosslinking of the layer of precursor molecules at least one of low energy electrons, x-ray radiation, β-radiation, γ-radiation, photons, ions or plasma can be applied.

The linker may be either aliphatic or comprise ethylene glycol moieties and may have a length between 0.5 and 10 nm.

The linker may further comprise ethylene glycol moieties and have a length between 0.5 and 3 nm.

It is further envisaged that the density of the amine groups of the CNMs of the third step described above is determined by the provision of a mixture of precursor monomers comprising amine groups or at least one of nitrile and nitro groups with monomers lacking these groups in the first method step above.

The precursor molecules provided in the first method step as described above may form a self-assembled molecular layer in the following step. The formed CNM in the third method step as described above may have a thickness of less than 5 nm.

The azide groups of the azide-terminated CNMs can be arranged in a defined pattern.

A further step comprising the coupling a receptor to the azide-terminated CNM may also be performed.

It is intended that remaining azide groups of the CNM after coupling a receptor, which are not coupled to the receptor, can be capped by providing an excess of azide-reactive groups; or converted to amine groups under reducing conditions.

Another object of the present disclosure is a CNM comprising amine groups coupled to a linker comprising functional azide groups, thus forming an azide-terminated CNM.

The density of the functional azide groups of such a CNM will be at least 10¹⁴ molecules per cm².

It is further intended that the azide-terminated CNM can be attached to a surface selected from the group comprising gold, silver, copper, aluminium, titanium, stainless steel, silicon, silicon oxide, silicon nitride, germanium, indium tin oxide, graphene, graphene oxide, glassy carbon, glass, polymers, ceramics.

The azide terminated CNM may further comprise a receptor that is covalently coupled to the azide-terminated CNM via a linker comprising a triazole moiety.

The density of the receptor molecules of a azide terminated CNM shall be at least 10¹⁰ molecules per cm², and the receptor molecule can be a synthetic or natural biopolymer selected from the group comprising oligonucleotide, peptides, polyketides, sugars or small molecules with a molecular weight below 900 dalton.

A further object of the present disclosure is a biosensor comprising an azide terminated CNM manufactured by a method as described above.

A method of the use of an azide terminated CNM manufactured by a method as described above for the detection of analytes in a biosensor is another object of the present disclosure.

A method of use of an azide terminated CNM manufactured by a method as described above in massive parallel sequencing is a further object of the present disclosure.

### Summary of the figures

The disclosure will now be described on the basis of figures. It will be understood that the embodiments and aspects of the disclosure described in the figures are only examples and do not limit the protective scope of the claims in any way. The invention is defined by the appended claims.

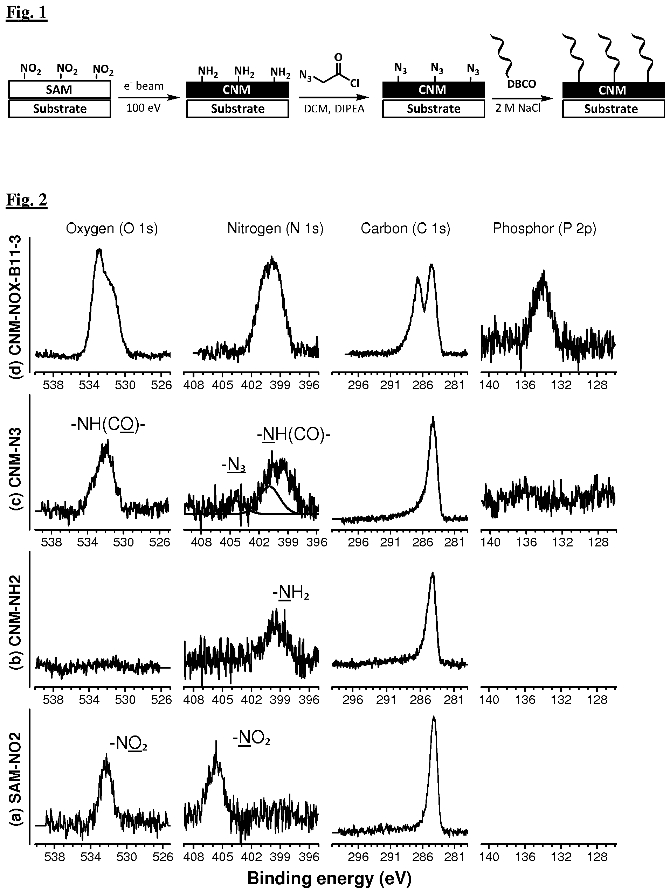

- Fig. 1: Fabrication of azide-terminated CNMs on gold substrate and immobilisation of DBCO-derivatised Spiegelmers.
- Fig. 2: XPS detail spectra for the steps of fabrication of azide-terminated CNMs on gold substrates and immobilisation of DBCO-derivatised Spiegelmer: (a) starting materials are nitro-terminated SAMs on a gold substrates with their characteristic singlet emissions for O at 532.0 eV, N at 406.5 eV and C Is peak at 284.5 eV; (b) when converting the SAM into a CNM, NO₂ is reduced to NH2 (almost no O, N is shifted to 400.0 eV) and the C Is emission is broadened; (c) after binding 2-azidoacetyl chloride, O is present again (amide group) and a N Is doublet appears at 405.0 and 401.8 eV (significant for azide groups); (d) immobilising NOX-B11-3-DBCO to the surface leads to a strong O and N doublet as well as a C triplet which are typical for nucleic acids; also the appearance of a P emission underlines the presence of Spiegelmer.

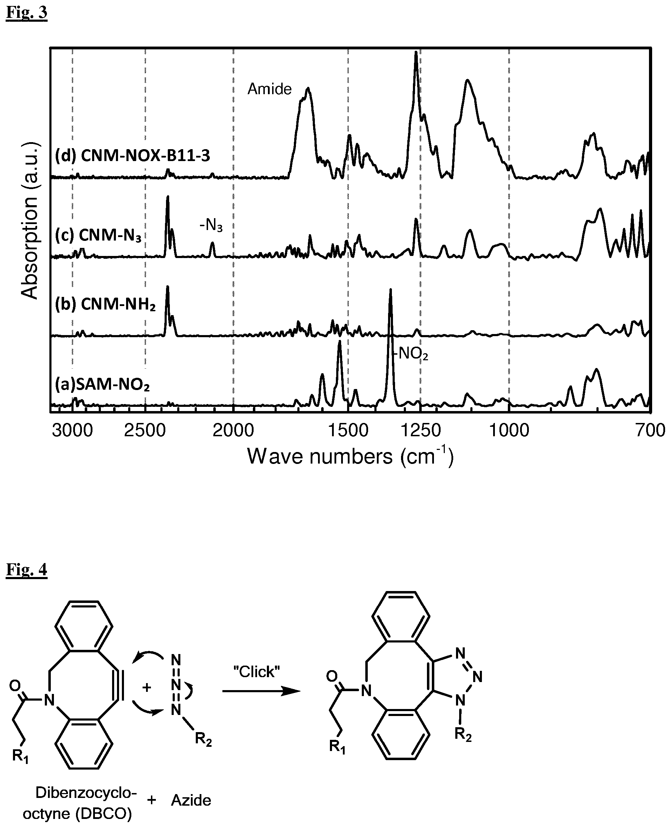

- Fig. 3: IRRAS spectra for the steps of fabrication of azide-terminated CNMs on gold substrates and immobilisation of DBCO-derivatised Spiegelmer. (a) The spectrum of the nitro-biphenyl-thiol (NBPT) self-assembled monolayer is dominated by nitro (1,551 cm⁻¹, 1,346 cm⁻¹ and 856 cm⁻¹) and aromatic bands (1,597 cm⁻¹, 1,531 cm⁻¹, 1,471 cm⁻¹); (b) the spectrum of the cross-linked carbon nanomembrane (CNM) shows almost no significant bands indicating the amorphous structure of the CNM (note the NH2 band is not visible); (c) after immersion in the azide-linker solution, a strong band at 2,110 cm⁻¹ indicates a successful linker coupling; (d) after Spiegelmer coupling, the azide band disappears and a strong amide band at 1750 cm⁻¹ can be seen.
- Fig. 4: Scheme of copper-free azide-alkyne click reaction. A strained cyclic alkyne (e.g. DBCO) attached to Ri (e.g. a sensitive biomolecule) reacts readily under mild condition with an azide which is bound to compound R₂ (CNM).

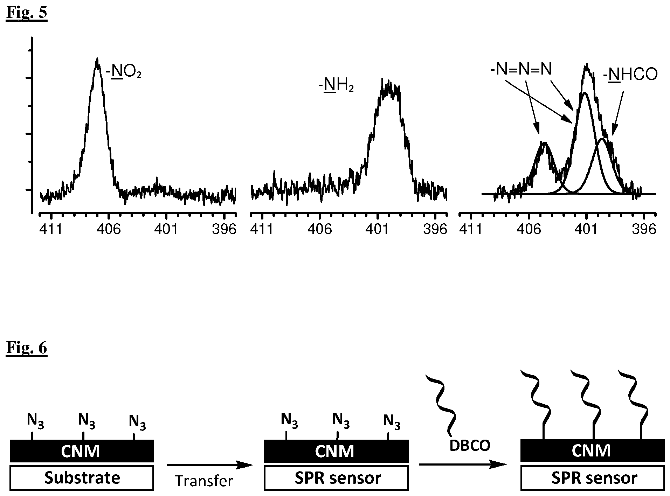

- Fig. 5: XPS nitrogen detail spectra for the steps of fabrication of azide-terminated CNMs on aluminium substrates.
- Fig. 6: Transfer of azide-terminated CNMs to SPR-sensor chips and immobilisation of DBCO-derivatised Spiegelmers.

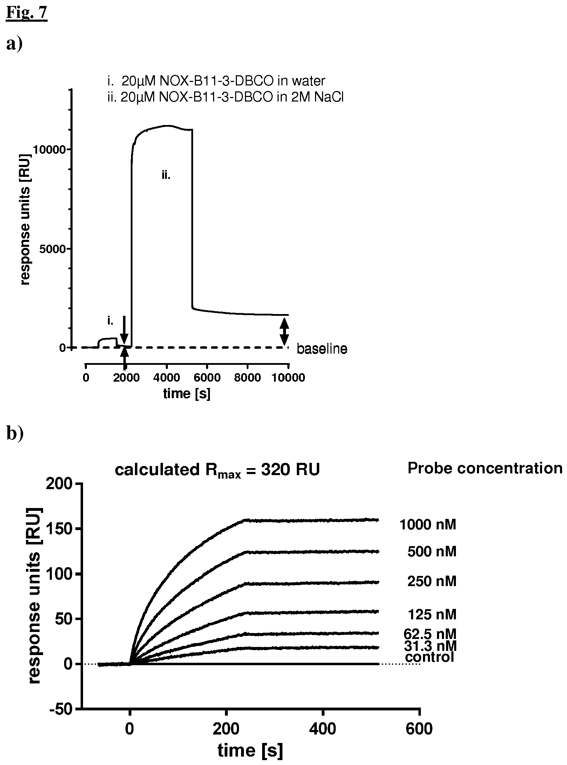

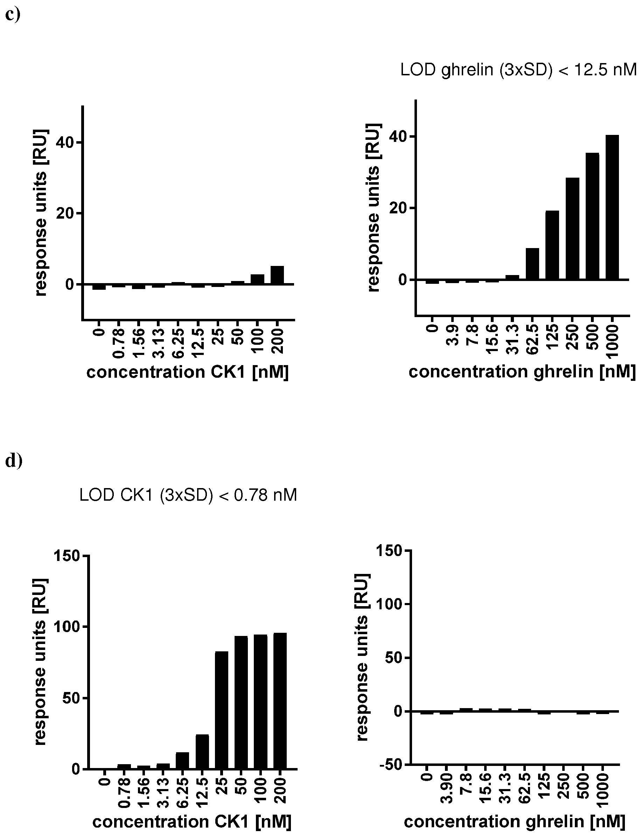

- Fig. 7: SPR measurements (on a Biacore SPR system) demonstrate the functionality of immobilised NOX-B11-3 on SPR-sensor chips with azide-terminated CNMs as functional interposer: (a) successful immobilisation of NOX-B11-3-DBCO to azide-terminated CNM leads to a pronounced SPR shift (corresponding to about 1,600 RU (response units)); (b) identification of a surface attached Spiegelmer NOX-B11-3 by specific hybridisation probe; the sensorgrams show a concentration-dependent behaviour for the hybridisation probe (0 - 1,000 nM/L) with a (calculated) maximum of 320 RU; (c) even human ghrelin (0 - 1000 nM) binding works and is saturable which confirms the functional activity of immobilised NOX-B11-3. On the same chip, injection of a human chemokine (CK1) did not lead to binding; (d) in a second experiment, CK1-binding DBCO-conjugated Spiegelmer was immobilised on a CNM-azide coated Biacore chip. Whereas CK1 gave a specific signal, ghrelin did not. This shows the high specificity of the system. Biacore conditions for ghrelin measurement: temperature: 25 °C, running buffer: Tris-HCl pH 7.4, 150 mM NaCl, 5 mM KCl, 1 mM CaCl₂, 1 mM MgCl₂. The report point, for which the values are plotted, is at the end of the dissociation phase (480 s after the start of the injection/after 240 s of dissociation). The data are referenced against a flow cell that was loaded with a 40 nt long control Spiegelmer of an arbitrary sequence.

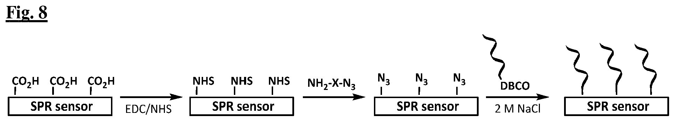

- Fig. 8: Immobilisation of DBCO-derivatised Spiegelmers on azide-terminated SPR-sensor chips based on commercially available carboxymethyl-dextran SPR-sensor chips. The carboxyl groups are activated by established EDC/NHS chemistry followed by coupling a short, bifunctional linker containing an amine and azide group.

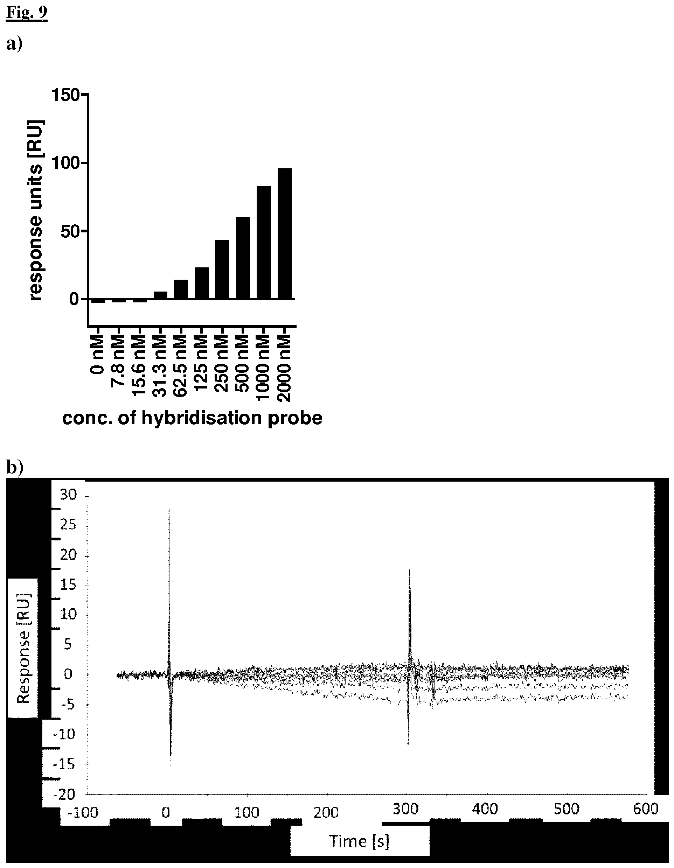

- Fig. 9: SPR measurements (on a Biacore SPR system) of NOX-B11-3 directly immobilised on a commercially available carboxymethyl-dextran SPR-sensor chip without a CNM as a functional interposer: (a) identification of a surface attached Spiegelmer NOX-B11-3 by specific hybridisation probe; the read-outs show a concentration-dependent behaviour; (b) sensorgrams after injection of a serial dilution of ghrelin (0 - 1000 nM). In contrast to NOX-B11-3 immobilised on an azide-terminated CNM (see Fig. 7), the directly immobilised Spiegelmer does not show an activity toward its specific target.

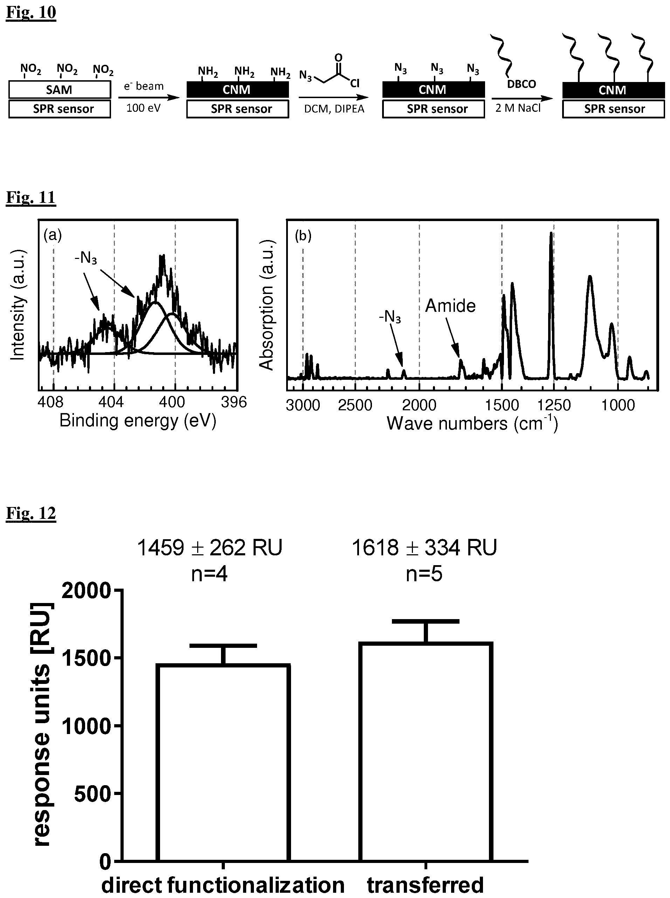

- Fig. 10: Fabrication of azide-terminated CNMs directly on SPR-sensor chips and immobilisation of DBCO-derivatised Spiegelmers.
- Fig. 11: Surface analysis of an azide-terminated CNM directly fabricated on SPR-sensor chips: (a) XPS detail spectrum of the nitrogen N-1s region. Three signals can be fitted to the raw spectrum. The typical duplet of the azide group at 404.5 and 401.5 eV demonstrates the presence of azide group and the remaining emission at 399.0 eV stems mostly from the amide linkage, (b) The corresponding IRRAS spectrum also confirms the coupling of the linker by characteristic bands at 2,110 cm⁻¹ (azide) and 1,720 cm⁻¹ (amide).
- Fig. 12: Immobilisation level in response units (RU) for SPM2-DBCO measured by SPR analysis on azide-terminated CNM directly fabricated on the SPR-sensor chip and on azide-terminated CNM transferred from another gold surface to the SPR-sensor chip.

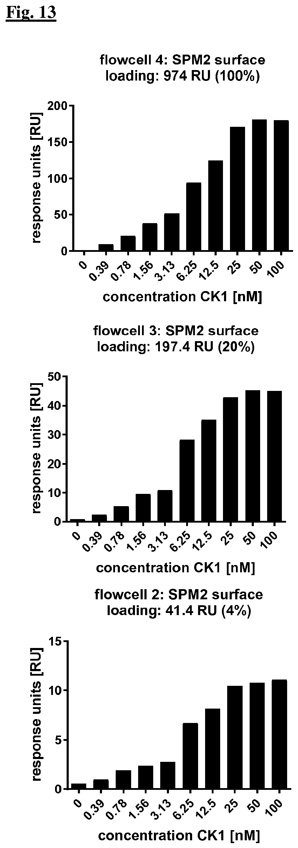

- Fig. 13: Read-out of the binding event of the chemokine CK1 to SPM2-DBCO, which was immobilised with different surface densities (measured in response units after immobilisation) on SPR-sensor chips with azide-terminated CNMs. The read-out is referenced to a control flow cell with the non-functional control Spiegelmer revSPM2-DBCO.

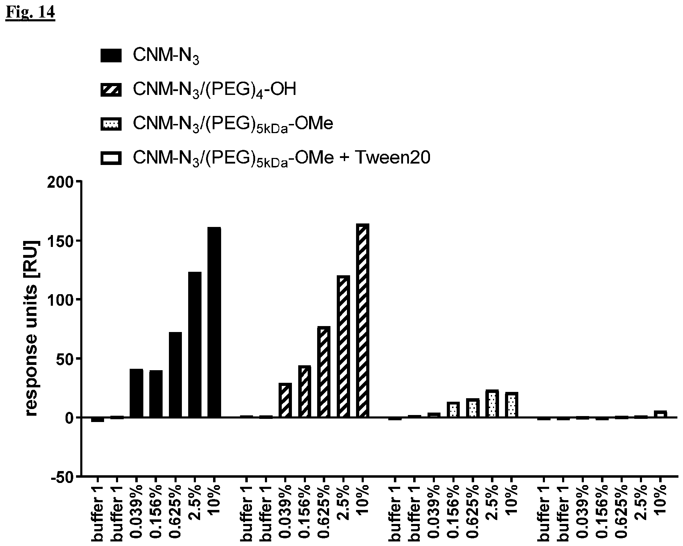

- Fig. 14: Reduction of the non-specific binding of swab/UTM to the azide-terminated CNM by PEGylation and addition of a surfactant to the running medium. Read-out of the binding event by report point 2 (at the end of the dissociation phase). Non-referenced data.

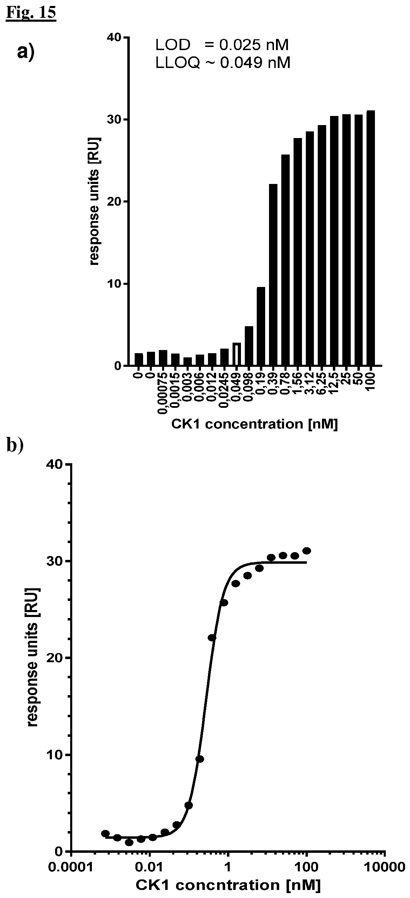

- Fig. 15: Binding of the chemokine CK1 to immobilised SPM2-DBCO in 100% swab/UTM under optimised assay conditions. Referenced as (FC2-FC1). (a) Read-out of the binding event by report point 2 (at the end of the dissociation phase), (b) The experimental data are fitted by a 4-parameter sigmoidal curve.

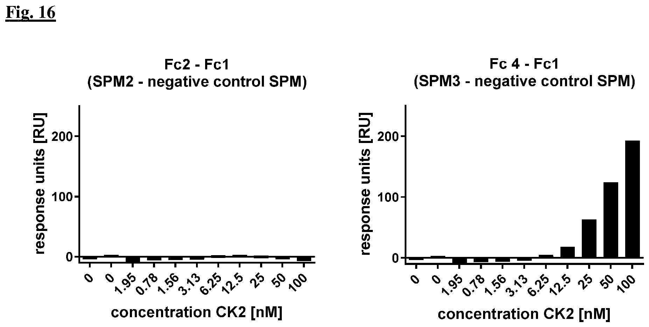

- Fig. 16: SPR measurements (on a Biacore SPR system) demonstrate the functionality of immobilised SPM3-DBCO on SPR-sensor chips with azide-terminated CNMs as functional interposer: Whereas CK2 gave a specific signal on SPM3-DBCO (Flowcell 4), it did not give a signal on the negative control Spiegelmer on Flow cell 2. Biacore conditions for CK2 measurement: temperature: 25 °C, running buffer: Tris-HCl pH 7.4, 150 mM NaCl, 5 mM KCl, 1 mM CaCl₂, 1 mM MgCl₂. The report point for which the values are plotted is at the end of the dissociation phase (480 s after the start of the injection/after 240 s of dissociation). The data are referenced against a flow cell loaded with a 40 nt long control Spiegelmer of an arbitrary sequence.

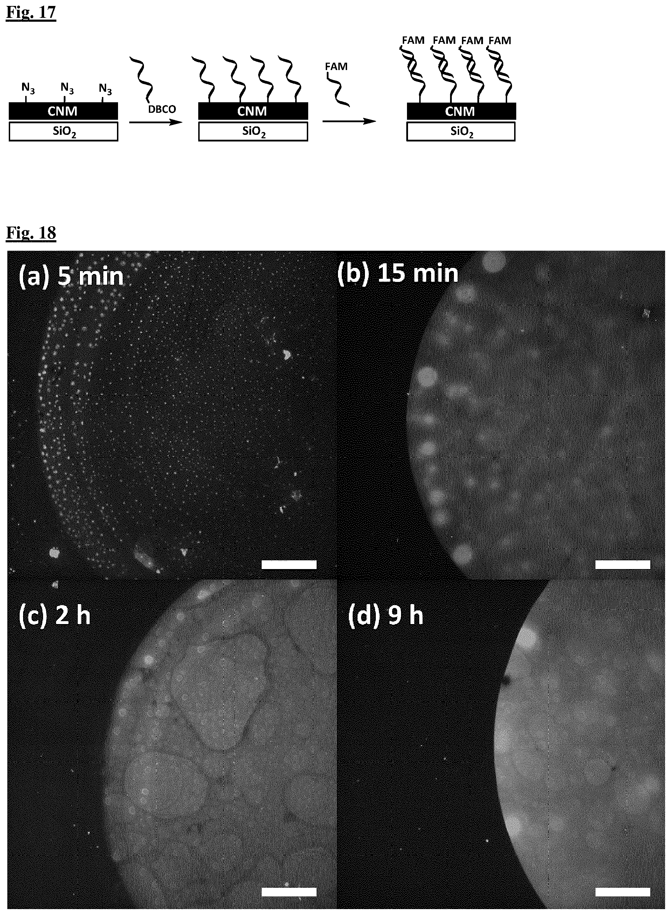

- Fig. 17: Scheme for detecting binding events by fluorescence microscopy. The Spiegelmer NOX-B11-3-DBCO is immobilised as droplets on an azide-terminated CNM, which was transferred onto an SiO₂ support. To make the Spiegelmer optically visible a fluorescently-labelled hybridisation probe is used.
- Fig. 18: Fluorescence signal of the fluorescently-labelled hybridisation probe after binding to the Spiegelmer NOX-B11-3-DBCO, which was immobilised to the azide-terminated CNM with different incubation times (given in the figures) corresponding to different surface densities.

### Detailed Description

An "oligonucleotide" designates in the context of the present disclosure single-stranded nucleic acids with a length of 4 to 100 nucleotides. Oligonucleotides may comprise modifications such as labels or reactive groups for covalent immobilisation, crosslinking or derivatisation i.e. hydroxyl, phosphatidyl, sulfonic ester, thiol, alkyne, strained cycloalkyne, azide, hydrazide, or EDC. Oligonucleotides may also comprise non-nucleoside linking moieties such as single unit or repeating ethylene glycol, e.g. triethylene glycol (TEG), hexaethylene glycol (HEG) or even polyethylene glycol (PEG), which can join subunits or regions of the oligonucleotide. Subunits may also be joined by non-covalent interactions such as base pairing and/or haptens and their binding molecule (such as biotin and avidin) to become functionally linked in an oligonucleotide. Oligonucleotides may hybridise to complementary sequences under suitable conditions. Unblocked 3'-ends of oligonucleotides may serve as primers for polymerases or as acceptors for another nucleic acid in a ligase reaction.

A "nucleic acid" shall be understood within the present disclosure as a polymer comprising nucleosides. Said polymer may comprise naturally occurring nucleosides (i.e. adenosine, thymidine, guanosine, cytidine, uridine, deoxyadenosine, deoxythymidine, deoxyguanosine and deoxycytidine), nucleoside analogues (i.e. 2-aminopurine, 2-aminoadenosine, 2-thiothymidine, inosine, pyrrolpyrimidine, 3-methyladenosine, 5-methylcytidine, C5-bromouridine, C5-fluorouridine, C5-iodouridine, C5-propynyluridine, C5-propynylcytidine, C5-methylcytidine, 7-deazaadenosine, 7-deazaguanosine, 8-oxoadenosine, 8-oxoguanosine, O(6)-methylguanine and 2-thiocytidine), chemically modified bases, biologically modifies bases (i.e., methylated bases), intercalated bases, abasic sites, ribose-sugars (RNA) including ribo-thymidine, 2'-deoxyribose sugars (DNA) including deoxyribo-uridine, terminal 3'-deoxyribose or 2',3'-dideoxyribose sugar, modified sugars (i.e., 2'-O-methylribose (2'-OMeRNA)), or modified phosphates (i.e., phosphorothioate and 5'-N-phosphoramidites). In addition, the backbone may be modified by synthetic sugars or analogues thereof. Non-limiting examples are so-called "locked" (LNA), "unlocked" (UNA), 2'-flouroribose (FNA), arabinose (ANA), 2'-flouroarabinose (FANA), hexose (HNA), threose (TNA), acyclic threose (aTNA), serinol (SNA), glycerol (GNA) sugar, "Spiegelmers" (L-form sugars), triazol-sugars (synthesised by a click-reaction) or a peptidic backbone (PNA) or combinations thereof.

The term "receptor" is used for a biomolecule which can bind to a given analyte. A receptor can be a biopolymer such as a peptide, a polysaccharide or polyketide, in particular a receptor is a (poly)peptide or a nucleic acid. A receptor may have a three-dimensional structure which allows a specific recognition of a cognate ligand. A nucleic acid receptor may recognise or interact with another complementary nucleic acid molecule and/or with a nucleic acid aptamer receptor or a given target molecule. The receptor may comprise a chemical group that can react with an azide group to form a stable covalent conjugate, preferably comprising a triazole group.

The present invention provides azide-terminated CNMs on a surface suitable for coupling of further substrates. An increased sensitivity for sensors based on surface interactions can be achieved by using the ultrathin carbon nanomembranes (CNMs) as intermediate layer (functionalisation interposer) between an active sensor surface for instance and a receptor moiety.

In contrast to the prior art the present invention refers to two-dimensional materials without bulk. Document US 2011/184196 A1 relates to carbon surfaces that may be carbon black, carbon fiber, carbon plates, carbon cloth, activated carbon, vitreous carbon, charcoal, activated charcoal, graphite powder, graphite fibers, carbon nanotubes, fullerenes, or combinations thereof. This document neither mentions nor anticipates the use of a two-dimensional material like graphene or as the present invention a CNM, which differs chemically from graphene.

It is to be noted that CNMs are not consisting exclusively of carbon. According to the description in paragraph 13 above, " Carbon Nanomembranes (CNMs) are novel two-dimensional (2D) carbon-based materials produced from radiation-induced crosslinking of a layer of precursor molecules with an aromatic molecular backbone...." The person skilled in the art knows that such a material does not only consist of carbon, but has at least H-atoms incorporated, as discussed in Turchanin et al., Langmuir 25, 7342 (2009). Moreover, additional functional groups as for examples thiol groups for CNMs produced from thiol-SAMs on Au-substrates (see also Turchanin et al., Langmuir 25, 7342 (2009)) can be implemented in a CNM. Especially amine-groups represent an inherent part of a CNM produced from precurors with nitro-groups as discussed in paragraph 16 above. In essence, a CNM does not represent a carbon surface as specified and modified in US 2011/184196 A1.

It is further to be noted that document US 2011/184196 A1 discloses a method of modifying a carbon surface, comprising modifying the carbon surface with a nitro-bearing group, reducing the nitro-bearing group to an amine-bearing group, and converting the amine-bearing group to an azide-bearing group by a diazo transfer reaction. In certain embodiments, the nitro-bearing group is a nitrophenyl group and the subsequent azide-bearing group is a phenyl azide group. An essential step of the method of document US 2011/184196 A1 is the modification of the carbon surface with a nitro-bearing group. This step is not necessary in the manufacturing of a functionalised CNM. The product after step a) and b) of claim 1 of the present invention, i.e. a layer of low-molecular weight aromatic precursor molecules, which comprise either amine groups or at least one of nitrile or nitro groups, is firstly not a carbon surface (see above) and secondly does not have to be modified with nitro-bearing (or other) groups. These groups are an inherent part of the CNM.

The CNM can be used to be functionalised with a wide range of different surfaces, but remains bound even under harsh conditions. A covalent attachment of receptors to the CNM guarantees the stability of the entire receptor-functionalised surface for repeated sensing cycles.

Click chemistry has been found to couple nucleic acids covalently and specifically to CNMs. Especially (copper-free) strain-promoted azide-alkyne cycloaddition provides very mild reaction conditions. Versatile azide-terminated CNMs are prepared directly on the sensor surface or via transfer of azide-terminated CNMs providing sensors ready for azide-alkyne cycloaddition. Reduction of unspecific background binding is achieved by immobilisation of polyethylene glycol (preferably (PEG)_{5kDa}) via click chemistry.

An advantage of using CNMs as interposer is that they are compatible with most surfaces used in biosensors. The CNM-functionalised surface is highly stable and durable with no fluorescence background. A further advantage is that CNMs can have a thickness of 0.3 to 30 nm, which is ideal for sensors based on surface-interactions. Azide-terminated CNMs have an advantageous long shelf life and can be functionalised with more sensitive biomolecule receptors rapidly before use as biosensor. It is beneficial that the azide-terminated CNMs are not influenced by buffer conditions such as pH, osmolarity, and electrolytes, which is optimal for biosensor applications in which the surface thickness and/or density is required to remain constant. The copper-free click chemistry is further a versatile and mild coupling chemistry that is compatible with very sensitive receptor molecules such as RNA. The functionalisation of the azide-terminated CNMs with oligonucleotides results advantageously in an ultra-high density of oligonucleotides with so far unprecedented functionality.

Azide-terminated CNMs are prepared by modifying amine-terminated CNMs with a short and highly reactive bifunctional linker containing a terminal azide group. It has been discovered that only very strong amine-reactive linkers, such as 2-azidoacetyl chloride, can be used to achieve a very high density of azide groups. The surface density of the azide groups can be controlled to some extent by the reaction time. Surface densities of more than 1 x 10¹⁴ cm⁻² are possible. More specifically, surface densities of more than 2 x 10¹⁴ cm⁻² are achieved. Yet weaker amine-reactive linkers can be used to achieve lower densities of azide groups as well. This makes the CNM ready for copper-catalysed as well as strain-promoted azide-alkyne cycloaddition (click chemistry). Even sensitive receptor biomolecules, such as proteins and oligonucleotides, react readily with this azide-terminated CNM under mild conditions without formation of undesired side products. Using this chemistry, alkyne-functionalised nucleic acids can be coupled to the azide-terminated CNM. A high receptor density allows the generation of highly sensitive biosensors. However, fine-tuning of the receptor density may allow optimisation for the given application.

The receptor nucleic acids to be immobilised can be single-stranded or double-stranded. They can be single-stranded or double-stranded oligonucleotides. The oligonucleotides can be aptamers. Such aptamers may also be in the non-natural L-configuration. In a special embodiment the oligonucleotides are Spiegelmers (L-aptamers).

The nucleic acid can be coupled by copper-free click chemistry. For this the oligonucleotide must be functionalised with a strained alkyne, e.g. dibenzocyclooctyl, DBCO; bicyclo[6.1.0]non-4-yne (BCN), and azadibenzocyclooctyne (ADIBO), preferably at a 5' or a 3' end. In particular, strained alkyne-modified aptamers can be coupled by this chemistry without losing their binding affinity to their targets.

It is also in the scope of this disclosure that the click chemistry may be used in the inverse geometry, i. e. that the CNM is modified with a linker containing at least one alkyne and the receptor is modified with at least one azide group.

Moreover, this approach is very versatile. Functionalised CNMs can be prepared on the final substrate directly if it is possible to form corresponding precursor SAMs on this substrate (e.g. gold, silicon). However, it is also possible to generate a CNM on a substrate other than the final substrate. This may be necessary if covalent bonding to the final substrate for SAM formation is not possible. In this case, after formation on one substrate, the CNM is transferred to the final substrate. The transfer can be done before or after each processing step, i.e. coupling of the bifunctional azide linker and coupling of the nucleic acids. Thereby, the combination of the CNM with the immobilisation chemistry for nucleic acids is universally applicable to various underlying surfaces. CNMs can be highly stable to retain their chemical and physical integrity even when transferred to a surface that has holes or other indentations that do not form contact with the membrane. This allows a greater flexibility with respect to surface formats that can otherwise not be coated with receptors. Another advantage of transfer of the CNM to the final biosensor surface is that not all materials are compatible with the crosslinking by irradiation and other synthesis steps including caustic solvents necessary to generate the final receptor functionalised CNMs.

In contrast to other chemistries, the click chemistry allows sequential immobilisation of nucleic acids and polyethylene glycol. This would not be straight-forward with several other surfaces, e.g. carboxyl-modified surfaces with labile activation groups (N-hydroxysuccinimide, NHS). NHS that has dissociated from the carboxyl groups, cannot be replaced once the nucleic acid has been coupled due to side reactions of the necessary activating agents (e.g. (1-ethyl-3-(3-dimethylaminopropyl)carbodiimide, EDC) with the amine groups of the nucleic acids.

To shield the parts of CNMs which are not covered by receptor molecules, the remaining azide groups can be further modified with the blocking polymer poly(ethylene glycol) (PEG) by using an alkyne-modified PEG, e.g. a DBCO-PEG conjugate. This suppresses the amount of unspecific adsorption and increases the limit of detection in sensor application with complex real-life samples. PEG could also have the potential of compromising the function of an immobilised aptamer in case it sterically hinders the free conformation of the aptamer molecules or the access of the targets to the aptamer. Therefore, a short DBCO-Tetraethylene glycol-OH was compared to a long DBCO-(PEG)_{5kDa}-OMe in terms of reducing unspecific binding and maintaining the aptamer activity.

Alternatives to PEG for biocompatibility and effective surface blocking are hydroxyethyl starch (HES), polypeptides such as peptides comprising Pro, Ala and Ser (PAS), and zwitterionic systems/polymers. It is preferred that the molecular weight of the blocking polymer correlates with the size of the receptor molecule so that the receptor remains accessible to interaction with its cognate ligand.

Density control of the surface functionalisation is also possible by using differently terminated precursor molecules, which can form a mixed-molecular SAM on a surface. After cross-linking, such a mixed SAM transforms into a CNM with mixed surface functions. [Turchanin and Gölzhäuser, Adv. Mater., 28 (2016) 6075]. The packing density and lateral order of the different precursor molecules in the parenting SAM determine the density of the functional groups of the resulting CNM.

Surprisingly, aptamers immobilised on azide-terminated CNMs retain a high target-binding activity. A person skilled in the art would have expected that inevitably remaining amine groups, that could not be derivatised with the azide linker, to interact with the negatively charged nucleic acid, thereby compromising its 3D-structure and affinity to its target. Primary amine groups (such as poly-lysine) are well known to lead to physisorption of nucleic acids. Likewise, hydrophobic interactions of the nucleobases with the CNM that originate from aromatic precursor molecules were expected to also inhibit the formation of the aptamers' 3D-structure. Given these expected limitations, the inventors surprisingly found that immobilised aptamers were still able to bind their targets with high affinity. Furthermore, the inventors surprisingly found that the orientation of azide and strained alkyne is important, since a DBCO-modified CNM showed higher protein adsorption than an azide-terminated CNM.

The possibility to immobilise nucleic acids in a functional conformation on surfaces allows for many applications, especially in sensor technology: Detection systems using this immobilisation scheme can be label-free (the analyte does not need be labelled) and could be based, e.g. on optical readout (fluorescence, absorption and luminescence), mass change (e.g., quartz crystal microbalance, microelectromechanical systems), refractory index (surface plasmon resonance, ring resonators, ellipsometry), charge (field effect transistors, electrochemical impedance), surface stress (cantilever biosensors) and atomic force microscopy.

Certain detection systems like those based on evanescent fields or field effect transistors require receptors that bind within a range of a few nanometres. Immobilised nucleic acids are advantageously surface-near due to the ultimate thinness of CNMs from below one nanometre up to 30 nm. This improves the sensitivity in sensor-applications. It is important that the surface itself does not cause any background for an efficient measurement of an analyte by an immobilised receptor. CNMs are highly crosslinked and do not swell or otherwise change mechanical properties by buffer changes thus providing a constant thickness. This is caused by the absence of charged molecules which could also influence the pH of the buffer and/or surface. Consequently, it turned out that CNMs are ideal for evanescent field, FET sensors and cantilever sensors which rely on an inert surface during measurement. Likewise, aptamers have a size of a few nanometres and are therefore within this range provided they are immobilised close to the surface.

The presence of azide groups after surface modification of CNMs was demonstrated by surface-sensitive techniques like X-ray photoelectron spectroscopy (XPS) and infrared reflection adsorption spectroscopy (IRRAS).

The maximum coverage is about 50% in respect to the originally present amine groups, which corresponds to a density of more than 2 x 10¹⁴ azide groups per cm². Thus, a very high density of oligonucleotides can be coupled to the membrane.

This extremely high density of immobilised and functional oligonucleotides on CNM allows an efficient generation of monoclonal clusters on a planar surface. Furthermore, the covalent nature of the attachment allows treatment under harsh conditions without loss of functionality. This is a prerequisite for massive parallel sequencing, especially if a narrow focal plane is to be achieved for very high content sequencing settings.

The template molecule can be amplified enzymatically, e.g. by bridge amplification, to generate a cluster of immobilised nucleic acid molecules composed of a single sequence. Today's sequencers can identify the sequence of many million clusters in parallel. Generally, the higher the density of clusters on a surface the more cost-effective is the sequencing reaction. Immobilised primer oligonucleotides as receptor molecules can hybridise to a complementary nucleic acid template to initiate enzymatic amplification. Bridge amplification can be used to generate randomly distributed clusters of nucleic acids on a surface bearing two different primer sequences. By providing an exceptionally high density of primer molecules on CNMs, less surface is needed to accommodate a sufficient amount of molecules for the detection of incorporated nucleotides. By reduction of the cluster size, more clusters can be accommodated on a surface. In addition, by providing a thin layer of membrane with immobilised receptors allows highly parallel detection of very small structures by a microscope without shifting the focus. This is in stark contrast to hydrogels normally used to immobilise primers to the surface of sequencing chips. Most other surface chemistries for immobilisation of oligonucleotides on a flat surface are not stable enough to resist repeated thermal denaturation steps needed to carry out bridge amplification.

Sequencing by synthesis mostly employs reversible terminators and fluorescent labels. After each sequencing cycle, the terminating groups and the labels are removed by a chemical cleavage step using strong reducing reagents such as TCEP (tris(2-carboxyethyl)phosphine). TCEP is known to convert azide groups into primary amines which could negatively affect the structure of the nucleic acid by surface interaction or background binding of nucleotides. Therefore, it is preferred to generate receptor-functionalised CNMs that are devoid of primary amine groups and any residual azide groups that could be converted by TCEP. As receptor molecules may comprise reactive amine groups, it is preferred to exclude any reactive amine groups before coupling the receptor to the CNM. For instance, all accessible reactive amine groups could be inactivated by capping with a monofunctional strong amine reactive linker comprising mostly polar, but not charged groups that do not interfere with the functionality of the receptor. Yet it is preferred to directly employ a strong amine reactive bifunctional linker such as 2-azidoacetyl chloride in order to functionalise these amine groups and achieve a high density of azide groups. As azide groups are not present in most receptors useful in biosensors such as oligonucleotides, residual azide groups can be removed safely after generation of a receptor-functionalised CNM without affecting the receptor. In order not to generate new amine groups by treatment with reducing reagents, it is preferred to cap the remaining unreacted azide groups with an excess of azide reactive compounds comprising an alkyne group, preferably a strained alkyne group. The azide-reactive capping compound preferably comprises polar, but not charged groups that do not interfere with the functionality of the receptor. Suitable capping compounds may also comprise blocking polymers such as (strained) alkyne-modified PEG.

In some applications, it may be necessary to cleave the receptor off the functionalised CNM. For instance, in the sequencing method of the Illumina sequencing platforms, one primer is cleaved selectively to remove one of the strands in order to carry out the sequencing on a single stranded nucleic acid. The cleavage can be achieved by enzymatic, chemical and physical means. It is preferred to cleave the linker region to remove the receptor molecule. Site selective cleavage of a linker is achieved by photocleavage using scissile groups such as nitrobenzyl. Otherwise chemical cleavage is effective and inexpensive using diol cleavable linker or linkers comprising disulfide bonds.

The distribution of azide groups on a membrane can be controlled by lithographic means. Especially photolithography allows a highly controlled and site-specific conversion of azide groups to amine or imine groups. These groups can thus be used for different coupling steps using amine-reactive compounds. It may be useful for some applications to passivate the amine groups with capping compounds. Preferred are blocking polymers that can prevent unspecific binding of analytes to the biosensor surface. The main advantage of photolithography is to introduce a defined patterning of features comprising azide groups on a surface that can be derivatised with different receptors and measured separately. Ideally, the features are isolated, non-overlapping patches of regular or irregular shape. Especially hexagonal shapes are useful to accommodate a high amount of features on a given surface with regular spaces in between.

It may also be possible to generate three-dimensional features by using a structured surface during the formation of crosslinked CNMs. Cavities are useful to allow reactions to occur separately in parallel. The cavities may be connected through channels in microfluidic devices to allow a regulated distribution of compounds and/or analytes. The azide-reactive receptor molecules may thus be immobilised at the surface of such cavities in a highly controlled fashion. A final microfluidic biosensor may thus allow the parallel detection of a multitude of analytes.

An important application of sensor surfaces with a high content of different features is massive parallel sequencing. For instance, DNA nanoballs as currently employed in the sequencer BGISEQ-500 (BGI Shenzen, China) can be immobilised separately on features with about 300 nm diameter. Alternatively, the features may comprise primer oligonucleotides as receptor molecules that can hybridise to a complementary nucleic acid template. The template molecule can be amplified enzymatically, e.g. by bridge amplification, to generate a cluster of immobilised nucleic acid molecules. If the amount of template molecules is low (i.e. one template in 10 features), only one template may be able to seed the amplification on the feature and will result in a monoclonal cluster for successful sequencing. By repeating the process of seeding and amplification all clusters are occupied and monoclonal. This approach allows the most cost effective sequencing of immobilised nucleic acids.

Nucleic acids, like Spiegelmers (L-(deoxy)ribonucleic acid aptamers with a strong affinity to a specific target) could be conjugated to strained alkyne DBCO to enable the copper-free click reaction. Following this concept, Spiegelmers were successfully immobilised to a surface (CNM) for the first time.

The success of Spiegelmer coupling to CNMs is clearly verified by XPS and IRRAS. Additionally, the maximum density of Spiegelmers on CNMs was roughly calculated to be 1 x 10¹³ Spiegelmers per cm². This corresponds to a coupling density of 5% in respect to the present azide groups or about 50% surface coverage (depending on the assumed shape of a Spiegelmer). Again, the coupling density is adjustable by the reaction time.

Measurements employing a flow-based surface plasmon resonance (SPR) showed an unexpectedly high activity of Spiegelmers immobilised on a CNM (transferred on a SPR-sensor chip). This was demonstrated on three different Spiegelmers. Each target (Ghrelin, and two chemokines) was detectable in the picomolar (pM) to nanomolar (nM) concentration range. It might be noted that these targets are very light proteins with molar masses of less than 10 kDa and, therefore, not easy to detect.

It is intended that a defined mixture of precursor monomers comprising nitro groups, nitrile groups or amine groups with monomers lacking these groups is provided prior to the generation of a CNM, thereby determining the density of amine groups prior to reaction with an amine-reactive linker comprising at least one azide group, thereby also determining the maximum density of functional azide groups of said generated CNM.

It is further intended that a defined mixture of precursor monomers comprising nitro groups, nitrile groups or amine groups with monomers lacking these groups is provided prior to the generation of CNM, wherein the ratio of precursors with the functional group versus precursors without the functional group in the mixture is preferably between 1:0.1 to 1:10,000, more preferably between 1:1 to 1:1,000 and most preferably between 1:1 and 1:100.

It is envisaged that the surface density of azide groups can be determined by reactivity of the amine-reactive group in the linker comprising the azide group.

The surface density of azide groups may be determined by concentration and/or duration of the reaction with the amine-reactive azide linker.

The surface density of azide groups may further be determined by mixture of the bifunctional amine-reactive linker comprising an azide group with an amine-reactive monofunctional linker, wherein said monofunctional linker preferably comprises substantially polar but uncharged groups.

The precursors can be provided as a self-assembled molecular monolayer on a surface prior to the generation of CNMs in order to limit resulting membranes to a thickness between 0.5 and 5 nm, more preferably between 1 and 2 nm.

At least one of the precursor molecules can be selected from the group consisting of phenyl, biphenyl, terphenyl, quaterphenyl, naphthaline, anthracene, bipyridine, terpyridine, thiophene, bithienyl, terthienyl, pyrrole and combinations thereof, wherein biphenyl is most preferred.

The surface for forming a self-assembled monolayer may be selected from the group consisting of gold, silver, titanium, zirconium, vanadium, chromium, manganese, cobalt, tungsten, molybdenum, platinum, aluminium, iron, steel, copper, nickel, silicon, germanium, indium phosphide, gallium arsenide, and oxides, nitrides or alloys or mixtures thereof, indiumtin oxide, glassy carbon, sapphire, and silicate or borate glasses and the groups for monolayer formation of the precursor monomers comprise groups for attachment to said selected surfaces.

The linker may comprise an amine-reactive group comprising acyl halides, esters, aldehyde, sulfonyl halides, isocyanate, isothiocyanate, carbodiimide, flourobenzene, imidoester, epoxide, wherein highly reactive groups are preferred comprising acyl halides, N-hydroxysuccinimide esters, imidoesters, epoxides, and most preferably acetyl chloride.

The distribution of azide groups on the surface can be controlled by photolithography, preferably by using an azide-terminated linker comprising a photo-activated amine-reactive group or more preferably by using a linker comprising a photo-cleavable group and most preferably by direct photolysis of the azide group of the amine-reactive linker to an amine or imine group. These may preferably be capped with a polar but uncharged amine reactive reagent in order to avoid interactions of the amine groups with the receptor, the analyte or the matrix in which the analyte is dissolved.

The invention relates further to a method for generating a receptor-functionalised CNM comprising the steps of
a) providing an azide-terminated CNM and a receptor molecule comprising an azide-reactive group,
b) coupling the receptor to the CNM under conditions compatible with the click reaction.

Remaining unreacted azide groups of the CNM after coupling of the receptor molecule can be capped by providing an excess of azide-reactive groups or converted to amine groups under reducing conditions.

The azide-reactive group may comprise an alkyne group, a strained alkyne group and or a dibenzocyclooctyl (DBCO), bicyclo[6.1.0]non-4-yne (BCN) or azadibenzocyclooctyne (ADIBO).

The azide-reactive group can be coupled to a terminus of a receptor biopolymer, preferably a 5' or a 3' end of a nucleic acid.

Remaining unreacted azide groups of the CNM after coupling of the receptor molecule can be capped by providing an excess of azide-reactive group comprising polymer selected from polyethylene glycol (PEG), hydroxyethyl starch (HES), polypeptides such as PAS, or zwitterionic systems/polymers, wherein PEG is most preferred.

The disclosure relates also to an azide-terminated CNM comprising amine groups coupled to a linker comprising functional azide groups.

The azide-terminated CNM can have a thickness in the range of 0.3 to 30 nm, or 0.5 to 5 nm or1 to 2 nm.

In another embodiment, the disclosure relates to an azide-terminated CNM comprising amine groups coupled to a linker comprising functional azide groups, wherein reactive primary amine groups are reduced by more than 80%, 90%, or 99%.

The linker molecule may comprise an azide group that is either aliphatic or comprises at least one ethylene glycol moiety and has a length of preferably 0.5 to 10 nm, and more preferably 0.5 to 3 nm.

The density of the functional azide groups shall be at least 10¹³ molecules per cm², more preferably more than 5 x 10¹³ molecules per cm², or more than 10¹⁴ molecules per cm².

The azide-terminated CNM may retain more than 80%, or 90% or 95% of functional azide groups after 2 weeks when stored under ambient conditions.

The linker comprising a terminal azide group may also comprise a cleavable group, wherein the cleavage can be achieved selectively under chemical and/or physical conditions or wherein the cleavable group is photocleavable and preferably comprises a nitrobenzyl group.

The CNM may be attached to a surface which is structurally patterned, wherein the pattern may consist of channels, rifts, pillars and wells.

The distribution of azide groups of azide-terminated CNM may be patterned and the pattern may comprise any geometrical pattern, including linear, circular, triangular, rectangular, and/or hexagonal shapes, wherein circular and hexagonal shapes are preferred.

The distribution of azide groups may be patterned and the features have an inner diameter of less than 1 cm, or less than 100 µm, or less than 5 µm, wherein the non-azide functionalised isolation between the features comprising the azide groups is less than 1 mm, preferably less than 10 µm and most preferably less than 500 nm.

In another embodiment, the azide-terminated CNM is characterised in that the membrane is substantially free, preferably floating on a liquid, most preferably floating on water and not attached to a surface.

In another embodiment, the azide-terminated CNM is characterised in that the free membrane is highly stable so that it can be transferred to a solid surface without affecting the structural and chemical integrity of the membrane.

The azide-terminated CNM may be characterised in that the free membrane is highly stable so that it can be transferred to a solid surface comprising holes, indentations or similar features in which the membrane is not attached without affecting the structural and chemical integrity of the membrane.

The azide-terminated CNM may further be characterised in that only one side of the membrane is azide-terminated, wherein said side of the membrane is substantially free of charged groups.

A receptor-functionalised CNM comprising surface-anchored amine groups coupled to a linker comprising a triazole moiety and a terminal receptor molecule is also within the scope of the present invention.

A receptor-functionalised CNM comprising surface-anchored amine groups coupled to a linker comprising a triazole moiety and a terminal receptor molecule, wherein reactive primary amine groups in the CNM membrane and linker are reduced as described above.

A receptor-functionalised CNM may further comprise a surface-anchored amine group coupled to a linker comprising a triazole moiety and a terminal receptor molecule, wherein reactive primary amine groups and azide groups in the CNM membrane and linker are absent.

The receptor-functionalised CNM may be characterised in that the membrane thickness is in the range of 0.3 to 30 nm, more preferably in the range of 0.5 to 5 nm, and most preferably in the range of 1 to 2 nm.

The receptor-functionalised CNM may be characterised in that the linker molecule connecting the receptor to the CNM comprising an amine and a triazole group is either aliphatic or comprises at least one ethylene glycol moiety and has a length of 0.5 to 10 nm, and more preferably 0.5 to 3 nm.

The receptor-functionalised CNM may further be characterised in that in addition to the receptor, blocking polymers are covalently attached to the membrane, wherein said polymeric molecules are selected from polyethylene glycol (PEG), hydroxyethyl starch (HES), polypeptides such as PAS, or zwitterionic systems/polymers, wherein PEG is the most preferred blocking polymer.

In addition to the receptor, blocking polymers can be covalently attached to the membrane by a linker comprising a triazole group.

In another embodiment, the receptor-functionalised CNM is characterised in that the molecular weight ratio of receptor to blocking polymers is in the range of 100:1 to 1:100, preferably 5:1 to 1:5, and most preferably 4:1 to 1:1.

In another embodiment, the receptor-functionalised CNM is characterised in that aptamer receptors are immobilised together with PEG, preferably with the molecular weight of 0.1 kDa to 50 kDa, more preferably 2 kDa to 20 kDa, most preferably 3 kDa to 10 kDa.

In another embodiment, the receptor-functionalised CNM is characterised in that the receptor molecule is a biopolymer, preferably a peptide or polypeptide, more preferably a nucleic acid and most preferably a nucleic acid aptamer.

In another embodiment, the receptor-functionalised CNM is characterised in that the receptor molecule is a nucleic acid that is coupled to the linker comprising a triazole group, preferably by the 5' and/or 3' end of a nucleic acid.

In another embodiment, the receptor-functionalised CNM is characterised in that the receptor molecule is a nucleic acid that is coupled by the 5' or 3' end to the linker comprising a triazole group.

In one embodiment, the receptor-functionalised CNM is characterised in that the receptor molecule is a nucleic acid aptamer, preferably a Spiegelmer.

In one embodiment, the receptor-functionalised CNM is characterised in that the receptor molecule is a nucleic acid, wherein the nucleic acid is coupled to the linker by the 5' end and the 3' end is not coupled and preferably unblocked.

In one embodiment, the receptor-functionalised CNM is characterised in that the receptor molecule is an oligonucleotide capable of priming the synthesis of a hybridised nucleic acid molecule in the presence of a polymerase.

In one embodiment, the receptor-functionalised CNM is characterised in that the receptor molecule is an oligonucleotide that remains covalently attached to the membrane even after repeated thermal denaturation steps.

In one embodiment, the receptor-functionalised CNM is characterised in that the density of the receptor molecules is at least 10¹⁰ molecules per cm², preferably more than 10¹¹ molecules per cm², and most preferably more than 5 x 10¹² molecules per cm².

In another embodiment, the receptor-functionalised CNM is characterised in that the linker comprising a triazole group also comprises a cleavable group, wherein the cleavage can be achieved selectively under enzymatic, chemical and/or physical conditions.

In another embodiment, the receptor-functionalised CNM is characterised in that the linker linking the receptor to the CNM comprises a triazole group and also comprises a cleavable group, wherein the cleavable group is photocleavable and preferably comprises a nitrobenzyl group.

In another embodiment, the receptor-functionalised CNM is characterised in that the membrane is attached to a surface which is structurally patterned, wherein the pattern may consist of channels, rifts, pillars and wells.

In another embodiment, the receptor-functionalised CNM is characterised in that the distribution of receptor molecules is patterned and the pattern comprises isolated features comprising azide groups, wherein the isolated features may have approximately linear, circular, oval, triangular, rectangular, and/or hexagonal shapes.

The distribution of receptor molecules may be patterned and the features have an inner diameter of less than 1 cm, or less than 100 µm, or less than 5 µm, wherein the area without receptor molecules between the features comprising the receptor molecules is less than 1 mm, preferably less than 10 µm and most preferably less than 500 nm.

The receptor-functionalised CNM of the present disclosure can be substantially free, preferably floating on a liquid, most preferably floating on water and not attached to a surface.

The receptor-functionalised CNM can be characterised in that the free membrane is highly stable so that it can be transferred to a solid surface without affecting the structural and chemical integrity of the membrane.

The receptor-functionalised CNM may be characterised in that the free membrane is highly stable so that it can be transferred to a solid surface comprising holes, indentations or similar features in which the membrane is not attached, without affecting the structural and chemical integrity of the membrane.

The invention relates also to the use of a receptor-functionalised CNM for detection of analytes in a biosensor, wherein the detection method of the biosensor is based on a solid surface and may comprise optical means comprising fluorescence, absorption and luminescence, or mass change, or refractory index change, or atomic force microscopy.

The receptor-functionalised CNM may be used for the detection of analytes in a biosensor and is characterised by reversible binding of said analytes to nucleic acid aptamer receptors.

The use of a receptor-functionalised CNM for detection of analytes in a biosensor can be characterised by enzymatic solid phase amplification of nucleic acids, wherein the receptor is an oligonucleotide and the amplification product is detected.

The use of a receptor-functionalised CNM for detection of analytes in a biosensor can be characterised by massive parallel sequencing of nucleic acids, wherein the nucleic acids are immobilised on isolated features and the biosensor is a sequencing device.

The use of a receptor-functionalised CNM for detection of analytes in a biosensor can be characterised by massive parallel sequencing of nucleic acids, wherein at least 10³, preferably 10⁶ and most preferably 10⁹ different nucleic acid templates are sequenced in parallel.

The use of a receptor-functionalised CNM for detection of analytes in a biosensor can be characterised by performing a ligase reaction of said analyte to the receptor thereby linking the analyte covalently to the receptor on the surface. This is applicable when both the receptor and the analyte are nucleic acids.

The use of a receptor-functionalised CNM for detection and/or sequencing of analytes in a biosensor can be characterised by performing a ligase reaction by specifically linking at least one labeled oligonucleotide to a complementary nucleic acid on the surface. This is applicable when said complementary nucleic acid is an analyte that is either captured by a receptor or previously amplified on the surface.

### Examples

### Example 1

Fabrication of azide-terminated CNMs on gold substrates and immobilisation of DBCO-derivatised Spiegelmers (Fig. 1)

Amine-terminated CNMs were prepared on gold thin films according to well-established protocols. [Eck et al. Adv. Mater., 12 (2000) 805] These CNMs were immersed into anhydrous dichloromethane under argon atmosphere followed by addition of N,N-diisopropylethylamine (DIPEA) obtaining 5 vol% DIPEA. The solution was cooled to 0 °C and one molar equivalent (in respect to DIPEA) 2-azidoacetyl chloride (30 vol% in dry diethyl ether) was added dropwise while stirring. The solution was brought to room temperature and stirred for at least 12 h. The reaction was terminated by quenching with ethanol. The substrates with the azide-terminated CNMs were rinsed with ethanol and dried in a nitrogen stream. They could be stored under ambient condition over weeks without decomposition of the azide group.

The presence of the azide group was confirmed spectroscopically by surface-sensitive techniques like X-ray photoelectron spectroscopy (XPS) and infrared reflection absorption spectroscopy (IRRAS). Each individual reaction step is plotted in Fig. 2 and Fig. 3. Starting from (a) nitro-terminated SAM via (b) cross-linked, amine-terminated CNM up to (c) azide-terminated CNM.

The activity of the azide groups was demonstrated by coupling a DBCO-conjugated Spiegelmer via copper-free click chemistry (Fig. 4). For the latter step, an azide-terminated CNM was immersed in 2 M NaCl with 10 µM DBCO-coupled Spiegelmer (NOX-B11-3-DBCO, for sequence and affinity information see Jarosch et al. Nucleic Acids Research, 34 (2006) e86) for 12 h. After rinsing extensively with 2 M NaCl and deionised water, the XPS-spectrum (Fig. 2d) as well as IRRAS-spectrum (Fig. 3d) clearly showed the successful Spiegelmer coupling.

### Example 2

### Fabrication of azide-terminated CNMs on aluminium substrates

Amine-terminated CNMs were prepared on aluminium substrates and subsequently functionalised with azide groups as described in Example 1. XPS was used to monitor the changes in the surface (Fig. 5). The spectra were acquired under comparable conditions, so the relative peak areas are a measure for the relative surface density of the respective elements. It can be seen that the surface density of azide groups is approximately 50% of that of the originally present amine groups.

### Example 3

Transfer of azide-terminated CNMs to SPR-sensor chips and immobilisation of ghrelin-detecting Spiegelmer NOX-B11-3-DBCO (Fig. 6)

Azide-terminated CNMs were prepared on gold substrates as described in Example 1. They were transferred successfully (without functional restriction of the azide groups) onto surface plasmon resonance (SPR) sensor chips using well-known transfer techniques for 2D-materials (e.g. US 8,377,243 B2).

The successful immobilisation of DBCO-derivatised Spiegelmers was detected by surface plasmon resonance (SPR). For that, ghrelin-binding Spiegelmer NOX-B11-3-DBCO was dissolved in water at 20 µM and passed over the SPR-sensor chip coated with an azide-terminated CNM in a commercial Biacore® 2000 system. Fig. 7a shows the in-situ recorded sensorgram. Injection of Spiegelmer in water led only to a small increase of about 50 RU (response units) corresponding to low amount of Spiegelmer coupled to the azide-terminated CNM. In contrast, when injecting the Spiegelmer in 2 M NaCl for 30 to 50 minutes the strong increase to more than 1,600 RU indicated a pronounced chemical binding of Spiegelmer to the azide-terminated CNM (Fig. 7a).

The presence and functional activity of the immobilised Spiegelmer was tested by injecting the complementary hybridisation probe (Fig. 7b) and, more importantly, ghrelin as the specific target of NOX-B11-3 (Fig. 7c). Both molecules were clearly detected by means of the immobilised Spiegelmer which clearly demonstrated the activity of such surface-bound aptamers. Ghrelin could be well quantified within the nanomolar range. Note again that this fact was not self-evident. On the same chip, injection of a human chemokine (CK1) did not lead to binding.

In a second experiment, the DBCO-derivatised Spiegelmer SPM2-DBCO (an L-aptamer designed to specifically bind a human chemokine (CK1)) was immobilised on a SPR-sensor chip coated with an azide-terminated CNM. Whereas the chemokine CK1 gave a specific signal, ghrelin did not. This shows the high specificity of the system.

### Example 4

Immobilisation of ghrelin-detecting Spiegelmer NOX-B11-3-DBCO on commercially available carboxymethyl-dextran SPR-sensor chips via conventional EDC/NHS chemistry (Fig. 8)

To emphasise the importance of a CNM as a functional interposer between sensor surface and aptamers as capture molecules, corresponding immobilisation experiments were carried out with NOX-B11-3 without a CNM as functional interposer. Commercially available SPR-sensor chips with carboxymethyl-dextran coating were activated according well-established NHS/EDC chemistry. The amine group of a short amine-azide linker reacted with the activated carboxyl groups obtaining an azide-terminated surface. Finally, NOX-B11-3-DBCO was immobilised to the azide groups just like to azide-terminated CNMs. To investigate the functionality of "CNM-free" immobilised NOX-B11-3 Spiegelmers, a hybridisation probe as well as human ghrelin were injected. Whereas the hybridisation probe indicated the presence (< 100 RU for 1,000 nM) of NOX-B11-3 on the chip (Fig. 9a), there was no activity towards ghrelin (Fig. 9b). These facts clearly illustrate the need for a CNM as functional interposer when immobilising aptamers.

### Example 5

Fabrication of azide-terminated CNMs directly on SPR-sensor chips and immobilisation of DBCO-derivatised Spiegelmers (Fig. 10)

Amine-terminated CNMs were directly grown on the gold-coated side of SPR-sensor chips via nitro-terminated precursor SAMs. These were converted to azide-terminated CNMs by using 2-azidoacetyl chloride as described in Example 1. Successful binding of the azide-linker was shown spectroscopically by XPS (Fig. 11a). Most significant is the detail spectrum of the nitrogen N Is region where three individual peaks can be fitted to the raw spectrum. The typical duplet of the azide group at 404.5 and 401.5 eV demonstrates the presence of the azide linker. The third emission at 399.0 eV stems mostly from amide linkage to the CNM. The corresponding IRRAS spectrum (Fig. 11b) also confirms the coupling of the linker by characteristic bands at 2,110 cm⁻¹ (azide) and 1,720 cm⁻¹ (amide). Other bands are artefacts (e.g. SiO₂ from the quartz support of the chip) because SPR-sensor chips are actually not designed for IRRAS.

In-situ immobilisation of the DBCO-derivatised Spiegelmer SPM2-DBCO as test probe was used to show that azide-terminated CNMs directly prepared on the surface of the SPR-sensor chips had no distinct difference compared to CNMs functionalised on their initial substrate and then transferred to the SPR-sensor chip (as shown in Example 3). Both chip preparations showed comparable maximal immobilisation levels of the SPM2-DBCO in the range of 1,500 ± 300 RU (response units) when Spiegelmer immobilisation was driven to the maximum (Fig. 12).

Based on a maximum immobilisation level of 1,800 RU that has been observed, the occupied surface area could be calculated.

The unit RU (termed resonance unit or response unit) is defined as 1 RU = 1 pg/mm², and is also often used to determine surface coverage. However, this description cannot be used ubiquitously. For instance, SPR measures the optical polarisability, size, and density of the molecules bound to the surface, which are related to but different from an SPR measurement in terms of mass per unit surface area. The polarisability depends on the wavelength of light, especially if the wavelength is close to the optical absorption band of the molecule (e.g. chromophores, UV-vis labels, etc.). Since most proteins have similar polarisabilities, the SPR signal may be considered approximately proportional to the coverage of molecules bound to the sensor surface, and pg/mm² is a useful way to quantify SPR sensitivity. (Source: https://www.sprpages.nl/component/phocadownload/category/1-download?download=12:biacalculation-manual-pdf - Downloaded 24 May 2017).

1,800 RU correspond therefore to 1.800 pg/mm² immobilised Spiegelmer SPM2-DBCO. Knowing the molar mass of SPM2-DBCO to be about 14,000 g/mol, this value corresponds to a surface density of 7.7 x 10¹² Spiegelmers per cm².

Assuming a spherical shape of the Spiegelmer and an approximate diameter of 3 nm for a Spiegelmer (based on the structures published in Oberthür et al., Nature Communications 6 (2015) 6923 and Yatime et al., Nature Communications 6 (2015) 6481, we can estimate the fraction of the surface area covered by Spiegelmers to be approximately 50 %.

### Example 6

Immobilisation of the Spiegelmer SPM2-DBCO on SPR-sensor chips coated with azide-terminated CNM

SPR-sensor chips coated with azide-terminated CNM were prepared as described in Example 5 and the Spiegelmer SPM2-DBCO was immobilised on them with different surface densities in the areas of different flow cells of a commercial Biacore system (to the maximum amount, and to about 1/5 as well as about 1/25 of this amount). For comparison, the non-functional Spiegelmer, revSPM2-DBCO, was immobilised to the maximal amount in the area of another flow cell. Injection of a concentration series of the target chemokine CK1 showed a dose-dependent binding to all flow cells. (Fig. 13)

### Example 7

Reduction of non-specific binding by passivation with polyethylene glycole

In order to reduce unspecific binding to the azide-terminated CNM surfaces, the approach for the passivation of the hydrophobic azide-terminated CNM surface by immobilisation of polyethylene glycol (PEG) as "shielding" molecule was tested. Reduction of the non-specific binding of a nasal swab from a healthy volunteer, dissolved in Universal Transport Medium (UTM by Copan), to the azide-terminated CNM was achieved by immobilisation of 5 kDa methoxy-terminated PEG-DBCO, "(PEG)_{5kDa}-OMe" but not by 4-unit-PEG, "PEG₄-OH". (Fig. 14). Addition of a non-ionic surfactant to the running medium further reduced the non-specific binding down to a level of less than 10 RU.

### Example 8

Optimised conditions and detection limits for the detection of the chemokine CK1 to immobilised SPM2-DBCO on azide-terminated CNM

The binding of the chemokine CK1 to the immobilised Spiegelmer SPM2-DBCO on an azide-terminated CNM surface was analysed under optimised conditions. The Biacore was set to a temperature of 25 °C. The physiological running buffer contained 0.1% Tween20. The Spiegelmer SPM2-DBCO was immobilised from 2 M NaCl solution with half-maximum surface density or higher to the area of flow cell 2 (FC2). The non-functional Spiegelmer revSPM2-DBCO was immobilised to a maximum surface density to the area of flow cell 1 (FC1) which served as reference flow cell. The surfaces were passivated by immobilisation of 5 kDa methoxy-terminated PEG-DBCO to the remaining azide groups after immobilisation of the Spiegelmers. Data was recorded as FC2-FC1 and the values at the report point after the dissociation were plotted. A concentration series of the chemokine CK1 was analysed in 100% swab/UTM with 0.1% (v/v) spiked-in Tween20.

The chemokine CK1 bound to the immobilised SPM2-DBCO with a limit of detection (LOD) of 0.025 nM (corresponding to baseline plus three times the standard deviation of the baseline, i.e. measuring unspecific binding of 100% swab/UTM without the chemokine CK1). The chemokine CK1 in 100% swap/UTM bound to the immobilised SPM2-DBCO with a half maximal effective concentration (EC₅₀) of 610 pM and a lower limit of quantification (LLOQ = baseline + 10 x SD of baseline) of 49 pM (Fig. 15).

### Example 9

Detection of the chemokine CK2 to immobilised SPM3-DBCO on azide-terminated CNM

The binding of the chemokine CK2 to the immobilised Spiegelmer SPM3-DBCO on an azide-terminated CNM surface was analysed under optimised conditions. The Biacore was set to a temperature of 25 °C. The physiological running buffer contained 0.1% Tween20. The Spiegelmer SPM3-DBCO was immobilised to a maximum surface density to the area of flow cell 4 (FC4). The Spiegelmer SPM2-DBCO was immobilised to a maximum surface density to the area of flow cell 2 (FC2). The non-functional Spiegelmer revSPM2-DBCO was immobilised to the area of flow cell 1 (FC1) which served as reference flow cell. The surfaces were passivated by immobilisation of 5 kDa methoxy-terminated PEG-DBCO to the remaining azide groups after immobilisation of the Spiegelmers. Data was recorded as FC4-FC1 and the values at the report point after the dissociation were plotted. A concentration series of the chemokine CK2 (0 - 100 nM) was analysed in 100% swab/UTM with 0.1% (v/v) spiked-in Tween20.

The chemokine CK2 bound to the immobilised SPM3-DBCO with a limit of detection (LOD) and lower limit of quantification (LLOQ) of 12.5 nM. CK2 did not give a specific signal on FC2, i.e. the flow cell with the anti-CKl Spiegelmer SPM2. (Fig. 16) This shows nicely the specificity and the applicability of this approach is for multiplexing purposes.

### Example 10

Detection of Spiegelmers immobilised on azide-terminated CNMs by fluorescein-labelled hybridisation probe

Fluorescence microscopy - often in combination with lithography - is another valuable technique to follow the Spiegelmer coupling and functional activity of immobilised Spiegelmers. As a model system, the ghrelin-binding Spiegelmer NOX-B11-3 as well as a specific, fluorescein-labelled hybridisation probe was used to make the Spiegelmer optically visible (Fig. 17). To avoid florescence quenching due to a metal surface close to the fluorophore, an azide-terminated CNM was transferred for example from the initial gold substrate to a silicon dioxide support (according to Example 3).

Patterns of immobilised NOX-B11-3 were obtained by applying the Spiegelmer as 10 µl droplets (10 µM NOX-B11-3-DBCO in 2 M NaCl) on a homogeneously azide-terminated CNM on SiO₂ (Fig. 18). After rinsing extensively (2 M NaCl and water), the sample was immersed for 30 min in 1 µM of the fluorescein labelled hybridisation probe and again properly rinsed. Using fluorescence microscopy, the shapes of the droplets were visualised whereas the areas without Spiegelmer remained dark.

By this droplet technique, a semi-quantitative analysis of the required time for the Spiegelmer-DBCO coupling to the azide-terminated CNM under non-flow conditions was possible in one experimental run. Droplets of 1 µM NOX-B11-3-DBCO were applied with incubation times between 5 minutes and 24 hours. After washing the sample properly with 2 M NaCl and water, it was immersed completely in 1 µM of the fluorescein labelled hybridisation probe for 3 min. Under the fluorescence microscope, a clear dependency of the brightness of the spots on the incubation time is obvious. Thus, it was confirmed, that the density of surface-attached Spiegelmer could be adjusted by incubation time and that this time should be at least 2 h under non-flow conditions when high densities are desired.

## Claims

1. A method for the manufacture of a functionalised carbon nanomembrane (CNM) comprising the steps of
a) Providing a surface and low-molecular weight aromatic precursor molecules comprising either amine groups or at least one of nitrile and nitro groups;
b) Applying a layer of the precursor molecules on the surface;
c) Crosslinking the layer of the precursor molecules in lateral direction by exposure to radiation for forming the CNM wherein nitrile or nitro groups, if present, are reduced to amine groups; and
d) Reacting the amine groups with a linker comprising at least one azide group resulting in an azide-terminated CNM

2. The method of claim 1, wherein the amine group of the CNM is reacted with the azide group-containing linker molecule by a strong amine-reactive group.

3. The method of any one of claims 1 or 2, wherein for crosslinking of the layer of precursor molecules at least one of low energy electrons, x-ray radiation, β-radiation, γ-radiation, photons, ions or plasma is applied.

4. The method of any one of claims 1 to 3, wherein the linker is either aliphatic or comprises ethylene glycol moieties and has a length between 0.5 and 10 nm.

5. The method of claim 3, wherein the linker comprises ethylene glycol moieties and has length between 0.5 and 3 nm.

6. The method of any one of claims 1 to 5, wherein the density of the amine groups of the CNMs of step c) is determined by the provision of a mixture of precursor monomers comprising amine groups or at least one of nitrile and nitro groups with monomers lacking these groups in step a).

7. The method of any of claims 1 to 6, wherein the precursor molecules provided in step a) are forming a self-assembled molecular layer in step b).

8. The method of any of claims 1 to 7, wherein the layer forming a CNM in step c) has a thickness of less than 5 nm.

9. The method of any of claims 1 to 8, wherein the azide groups of the azide-terminated CNMs are arranged in a defined pattern.

10. The method of any one of claims 1 to 9, further comprising the step of coupling a receptor to the azide-terminated CNM

11. The method of claim 10, wherein remaining azide groups of the CNM after coupling a receptor, which are not coupled to the receptor, are
i. capped by providing an excess of azide-reactive groups; or
ii. converted to amine groups under reducing conditions.

12. A carbon nanomembrane (CNM) comprising amine groups coupled to a linker comprising functional azide groups, thus forming an azide-terminated CNM

13. The azide-terminated CNM of claim 12, wherein the density of the functional azide groups is at least 10¹⁴ molecules per cm².

14. The azide-terminated CNM of any one of claims 12 or 13, wherein the azide-terminated CNM is attached to a surface selected from the group comprising gold, silver, copper, aluminium, titanium, stainless steel, silicon, silicon oxide, silicon nitride, germanium, indium tin oxide, graphene, graphene oxide, glassy carbon, glass, polymers, ceramics.

15. The azide terminated CNM of any one of claims 12 to 14, further comprising a receptor covalently coupled to the azide-terminated CNM via a linker comprising a triazole moiety.

16. The azide terminated CNM of claim 15, wherein the density of the receptor molecules is at least 10¹⁰ molecules per cm².

17. The azide terminated CNM of any one of claims 15 or 16, wherein the receptor molecule is a synthetic or natural biopolymer selected from the group comprising oligonucleotide, peptides, polyketides, sugars or small molecules having a molecular weight below 900 daltons.

18. A biosensor comprising an azide terminated CNM manufactured by a method of any one of claims 1 to 11.

19. Use of an azide terminated CNM manufactured by a method of any one of claims 1 to 11 for the detection of analytes in a biosensor.

20. Use of an azide terminated CNM manufactured by a method of any one of claims 1 to 11 in parallel sequencing.

## Patentansprüche

1. Verfahren zur Herstellung einer funktionalisierten Kohlenstoff-Nanomembran (CNM) umfassend folgende Schritte
a) Bereitstellen einer Oberfläche und niedermolekularer aromatischer Vorläufermoleküle, umfassend entweder Amingruppen oder mindestens eine von Nitril- und Nitrogruppen;
b) Aufbringen einer Schicht der Vorläufermoleküle auf die Oberfläche;
c) Vernetzen der Schicht der Vorläufermoleküle in seitlicher Richtung durch Bestrahlung zur Bildung der CNM, bei welcher Nitril- oder Nitrogruppen, falls vorhanden, zu Amingruppen reduziert werden; und
d) Umsetzen der Amingruppen mit einem Linker, umfassend mindestens eine Azidgruppe, resultierend in einer azidterminierten CNM

2. Verfahren nach Anspruch 1, bei welchem die Amingruppe der CNM mit dem azidgruppenhaltigen Linkermolekül durch eine starke aminreaktive Gruppe umgesetzt wird.

3. Verfahren nach einem der Ansprüche 1 oder 2, bei welchem zur Vernetzung der Schicht aus Vorläufermolekülen mindestens eines von niederenergetischen Elektronen, Röntgenstrahlung, β-Strahlung, γ-Strahlung, Photonen, Ionen oder Plasma bewirkt wird.

4. Verfahren nach einem der Ansprüche 1 bis 3, bei welchem der Linker entweder aliphatisch ist oder Ethylenglykol-Anteile umfasst und eine Länge zwischen 0,5 und 10 nm aufweist.

5. Verfahren nach Anspruch 3, bei welchem der Linker Ethylenglykolanteile umfasst und eine Länge zwischen 0,5 und 3 nm aufweist.

6. Verfahren nach einem der Ansprüche 1 bis 5, bei welchem die Dichte der Amingruppen der CNMs von Schritt c) durch die Bereitstellung einer Mischung von Vorläufermonomeren, umfassend Amingruppen oder mindestens eine von Nitril-und Nitrogruppen, mit Monomeren, bei welchen diese Gruppen in Schritt a) fehlen, bestimmt wird.

7. Verfahren nach einem der Ansprüche 1 bis 6, bei welchem die in Schritt a) bereitgestellten Vorläufermoleküle in Schritt b) eine selbstorganisierte Molekularschicht bilden.

8. Verfahren nach einem der Ansprüche 1 bis 7, bei welchem die in Schritt c) eine CNM bildende Schicht eine Dicke von weniger als 5 nm aufweist.

9. Verfahren nach einem der Ansprüche 1 bis 8, bei welchem die Azidgruppen der azidterminierten CNMs in einem definierten Muster angeordnet sind.

10. Verfahren nach einem der Ansprüche 1 bis 9, ferner umfassend den Schritt der Kopplung eines Rezeptors an die azidterminierte CNM

11. Verfahren nach Anspruch 10, bei welchem verbleibende Azidgruppen der CNM nach dem Koppeln eines Rezeptors, welche nicht an den Rezeptor gekoppelt sind,
i. durch Bereitstellen eines Überschusses an azidreaktiven Gruppen gekappt werden; oder
ii. unter Reduktionsbedingungen in Amingruppen umgewandelt werden.

12. Kohlenstoff-Nanomembran (CNM), umfassend Amingruppen, gekoppelt an einem Linker, umfassend funktionale Azidgruppen, wodurch eine azidterminierte CNM gebildet wird.

13. Azidterminierte CNM nach Anspruch 12, bei welcher die Dichte der funktionalen Azidgruppen mindestens 10¹⁴ Moleküle pro cm² beträgt.

14. Azidterminierte CNM nach einem der Ansprüche 12 oder 13, bei welcher die azidterminierte CNM an einer Oberfläche angebracht ist, ausgewählt aus der Gruppe umfassend Gold, Silber, Kupfer, Aluminium, Titan, Edelstahl, Silizium, Siliziumoxid, Siliziumnitrid, Germanium, Indiumzinnoxid, Graphen, Graphenoxid, Glaskohlenstoff, Glas, Polymere, Keramiken.

15. Azidterminierte CNM nach einem der Ansprüche 12 bis 14, ferner umfassend einen Rezeptor, kovalent gekoppelt an die azidterminierte CNM über einen Linker, umfassend eine Triazoleinheit.

16. Azidterminierte CNM nach Anspruch 15, bei welcher die Dichte der Rezeptormoleküle mindestens 10¹⁰ Molekülen pro cm² beträgt.

17. Azidterminierte CNM nach einem der Ansprüche 15 oder 16, bei welcher das Rezeptormolekül ein synthetisches oder natürliches Biopolymer ist, ausgewählt aus der Gruppe umfassend Oligonukleotide, Peptide, Polyketide, Zucker oder kleine Moleküle mit einem Molekulargewicht unter 900 Daltons.

18. Biosensor, umfassend eine azidterminierte CNM, hergestellt nach einem Verfahren gemäß einem der Ansprüche 1 bis 11.

19. Verwendung einer azidterminierten CNM, hergestellt nach einem Verfahren gemäß einem der Ansprüche 1 bis 11 zum Erfassen von Analyten in einem Biosensor.

20. Verwendung einer azidterminierten CNM, hergestellt nach einem Verfahren gemäß einem der Ansprüche 1 bis 11 in Parallelsequenzierung.

## Revendications

1. Procédé de fabrication d'une nano-membrane de carbone (CNM) fonctionnalisée comprenant les étapes suivantes
a) fourniture d'une surface et de molécules de précurseurs aromatiques de faible poids moléculaire comprenant soit des groupes amine, soit au moins un parmi les groupes nitrile et nitro ;
b) application d'une couche des molécules de précurseurs sur la surface ;
c) réticulation de la couche de molécules de précurseurs dans la direction latérale par exposition à un rayonnement pour former la CNM dans lequel les groupes nitrile ou nitro, s'ils sont présents, sont réduits en groupes aminé ; et
d) réaction des groupes amine avec un lieur comprenant au moins un groupe azide, résultant en une CNM à terminaison azide.

2. Procédé selon la revendication 1, dans lequel le groupe amine de la CNM est mis en réaction avec la molécule de lieur contenant un groupe azide par un groupe réactif aux amines fortes.

3. Procédé selon l'une quelconque des revendications 1 ou 2, dans lequel, pour la réticulation de la couche de molécules de précurseurs, au moins l'un parmi des électrons de faible énergie, un rayonnement de rayons X, un rayonnement β, un rayonnement y, des photons, des ions ou un plasma est appliqué.

4. Procédé selon l'une quelconque des revendications 1 à 3, dans lequel le lieur est soit aliphatique, soit comprend des fragments d'éthylène glycol et a une longueur comprise entre 0,5 et 10 nm.

5. Procédé selon la revendication 3, dans lequel le lieur comprend des fragments d'éthylène glycol et a une longueur comprise entre 0,5 et 3 nm.

6. Procédé selon l'une quelconque des revendications 1 à 5, dans lequel la densité des groupes amine des CNMs de l'étape c) est déterminée par la fourniture d'un mélange de monomères précurseurs comprenant des groupes amine ou au moins un parmi des groupes nitrile et nitro avec des monomères dépourvus de ces groupes dans l'étape a).

7. Procédé selon l'une quelconque des revendications 1 à 6, dans lequel les molécules de précurseurs fournies à l'étape a) forment une couche moléculaire auto-assemblée à l'étape b).

8. Procédé selon l'une quelconque des revendications 1 à 7, dans lequel la couche formant une CNM à l'étape c) a une épaisseur inférieure à 5 nm.

9. Procédé selon l'une quelconque des revendications 1 à 8, dans lequel les groupes azides des CNMs à terminaison azide sont disposés selon un motif défini.

10. Procédé selon l'une quelconque des revendications 1 à 9, comprenant en outre l'étape de couplage d'un récepteur à la CNM à terminaison azide.

11. Procédé selon l'une quelconque des revendications 1 à 10, dans lequel les groupes azides restants de la CNM après couplage d'un récepteur, qui ne sont pas couplés au récepteur, sont
i. coiffés en fournissant un excès de groupes réactifs aux azides ; ou
ii. convertis en groupes amine dans des conditions réductrices.

12. Nano-membrane de carbone (CNM) comprenant des groupes amine couplés à un lieur comprenant des groupes azide fonctionnels, formant ainsi une CNM à terminaison azide.

13. CNM à terminaison azide selon la revendication 12, dans laquelle la densité des groupes azide fonctionnels est d'au moins 10¹⁴ molécules par cm².

14. CNM à terminaison azide selon l'une quelconque des revendications 12 ou 13, dans laquelle la CNM à terminaison azide est fixée à une surface choisie dans le groupe comprenant l'or, l'argent, le cuivre, l'aluminium, le titane, l'acier inoxydable, le silicium, l'oxyde de silicium, le nitrure de silicium, le germanium, l'oxyde d'indium et d'étain, le graphène, l'oxyde de graphène, le carbone vitreux, le verre, les polymères, les céramiques.

15. CNM à terminaison azide selon l'une quelconque des revendications 12 à 14, comprenant en outre un récepteur couplé de manière covalente à la CNM à terminaison azide via un lieur comprenant un fragment triazole.

16. CNM à terminaison azide selon la revendication 15, dans laquelle la densité des molécules de récepteur est d'au moins 10¹⁰ molécules par cm².

17. CNM à terminaison azide selon l'une quelconque des revendications 15 ou 16, dans laquelle la molécule de récepteur est un biopolymère synthétique ou naturel choisi dans le groupe comprenant les oligonucléotides, les peptides, les polykétides, les sucres ou les petites molécules ayant un poids moléculaire inférieur à 900 daltons.

18. Biocapteur comprenant une CNM à terminaison azide fabriquée par un procédé selon l'une quelconque des revendications 1 à 11.

19. Utilisation d'une CNM à terminaison azide fabriquée par un procédé selon l'une quelconque des revendications 1 à 11 pour la détection d'analytes dans un biocapteur.

20. Utilisation d'une CNM à terminaison azide fabriquée par un procédé selon l'une quelconque des revendications 1 à 11 dans un séquençage parallèle.
